# EUROPEAN PATENT APPLICATION

(11) **EP 1 829 479 A1**
(43) Date of publication of application: **05.09.2007**
(21) Application number: 06713759.6
(22) Date of filing: 15.02.2006
(51) Int. Cl.: A61B 5/117, G06T 1/00, G06T 7/00

(54) **BIOMETRIC DISCRIMINATION DEVICE, AUTHENTICATION DEVICE, AND BIOMETRIC DISCRIMINATION METHOD**

(30) Priority: 16.02.2005 JP 2005038825
(71) Applicant: MATSUSHITA ELECTRIC INDUSTRIAL CO., LTD., Osaka 571-8501 (JP)
(72) Inventor: TSUKAHARA, Shinichi Matushita Electric Ind. Co. Ltd., Osaka 540-6319 (JP)
(74) Representative: Grünecker, Kinkeldey, Stockmair & Schwanhäusser Anwaltssozietät
(86) International application number: PCT/JP2006/302618
(87) International publication number: WO 2006/088042

(57) **Abstract**

An authentication system includes the following elements: a guide mirror to be used when a subject checks the position of the eye with an image reflected therefrom during imaging the eye of the subject; a lens disposed behind the mirror; and illuminating LEDs that are disposed symmetrically about the optical axis and can be switched for emitting near infrared rays. With this structure, the authentication system takes a plurality of images of the eye of the subject irradiated with illuminating rays from the illuminating LEDs at different lighting angles to the eye of the subject, determines whether the taken eye images are of a living eye or a forgery, and authenticates the iris.

## Description

### TECHNICAL FIELD

The present invention relates to a biometric discrimination system, authentication system, and biometric discrimination method.

### BACKGROUND ART

In recent years, various kinds of authentication methods using biometric information have been put to practical use, as a method of personal authentication during access to systems requiring high security, e.g. an entrance and exit control system, and an information system for storing such important information as personal data. The biometric information includes information specific to a subject, such as the fingerprints, iris, fundus blood vessels, features of the face, and vessel patterns of an arm or hand of a human body.

Among these methods, an authentication method using different wrinkle patterns in the iris portion of an eye (hereinafter referred to as "iris authentication method") is proposed and put to practical use in equipment requiring especially high security, because the method has high reliability, such as high personal identification rates and low false acceptance rates.

This iris authentication method includes: taking an image of the area including an eye of a subject (hereinafter referred to as "eye image"); coding the iris area of the eye image to represent the difference in the wrinkle patterns of the iris portion as numerical information; and providing authentication information. As disclosed in Japanese Patent No. 3307936, this authentication information is compared with the authentication information that has been registered (hereinafter referred to as "registered authentication information"). When the two pieces of information are determined to match with each other, the subject is authenticated as an already registered person. Because the iris images are most vivid when taken in the wave range of near infrared light, many of the apparatus used to take eye images have a visible-light cutting filter attached to the lens.

Such a method of authenticating an iris is widely put to practical use and gives excellent advantages as an authentication method to be used in places requiring high security, because the method provides high reliability, including low false rejection rates and low false acceptance rates.

On the other hand, in the above iris authentication method, there is a possibility that a person attempting to take an illegal action (hereinafter simply referred to as "unauthorized person") makes "false pretense" to be a registered person. Now, this "false pretense" means that an "unauthorized person" takes an image of the iris pattern of a registered person, and creates a photo, contact lens, or artificial eye having the iris pattern printed thereon (hereinafter referred to as "forgery"). Then, the unauthorized person pretends to be the registered person by using an unauthorized eye image of the forgery as the eye image of the registered person, to acquire authentication or registration of the unauthorized eye image of the forgery.

To address such a problem of false pretense using a forgery, Japanese Patent Unexamined Publication No. 2000-33080, for example, discloses the following iris authentication method. An illuminating unit for emitting brightness-adjustable visible light is provided. Changing the brightness of the illuminating light causes a miosis (a biological reaction in which the diameter of the pupil decreases on perception of bright light) in the eye of a subject. Detection of actual occurrence of a miosis determines whether or not the eye image is of a living body, and prevents false pretense.

However, the latter iris authentication method requires placing the subject in an environment with dark ambient light and causing a mydriasis (a biological reaction in which the diameter of the eye increases in dark ambient light) in the eye of the subject beforehand so that the subject has a miosis. For this reason, in an imaging apparatus using such an iris authentication method, the surrounding of the imaging apparatus must be kept in a dark environment, and thus the installation site is restricted.

### SUMMARY OF THE INVENTION

A biometric discrimination system includes the following elements: an illuminating unit for irradiating an area including an eye of a subject with illuminating rays; an imaging unit for taking a plurality of images of the eye irradiated with the illuminating rays at different lighting angles to the eye of the subject; a detector for detecting brightness of the iris in the taken eye images; and a determining part for comparing the detected brightness of the iris between the plurality of eye images and determining whether the subject is a living body or not according to the comparison results.

With this structure, a plurality of eye images are taken under illuminating rays at different lighting angels to an eye of a subject from the illuminating unit. Comparison of brightness between the plurality of taken eye images allows the determination of whether the taken eye images are of a living body or a forgery.

A biometric discrimination system includes the following elements: an illuminating unit for irradiating an eye of a subject with at least two illuminating rays at the same time in different radiation directions at different lighting angles to the eye of the subject; an imaging unit for imaging the eye of the subject irradiated with the illuminating rays; a detector for detecting brightness of at least two different areas in the iris in the taken eye image that are set according to the radiation directions of the illuminating rays to the eye of the subject; and a determining part for comparing the detected brightness between the at least two different areas in the iris. Whether the subject is a living body or not may be determined by the results of comparison in the determining part.

With this structure, an eye image can be taken while an eye of a subject is irradiated with at least two illuminating rays in different radiation directions at different lighting angles to the eye of the subject. Thus, even one eye image taken allow determination of whether the eye image is of a living body or a forgery.

A method of discriminating a living body includes: changing the lighting angles of illuminating rays from a illuminating unit to an eye of a subject; imaging the eye of the subject irradiated with the illuminating rays at different lighting angles; detecting the brightness of the iris and the brightness of the sclera in the eye images taken; calculating the ratios between the detected brightness of the iris and the detected brightness of the sclera; comparing the calculated brightness ratios between the plurality of eye images; and determining whether or not the subject is of a living body, according to the comparison results.

With this method, a plurality of eye images are taken under illuminating rays at different lighting angles to an eye of a subject, and comparison of the brightness ratios between the iris and the sclera in the plurality of taken eye images of the subject can determine whether the taken eye images are of a living body or a forgery.

A biometric discrimination method includes: irradiating an eye of a subject with at least two illuminating rays in different radiation directions at different lighting angles at the same time; imaging the eye of the subject irradiated with the illuminating rays; detecting the brightness of at least two different areas in the iris of the taken eye image that are set according to the radiation directions of the illuminating rays to the eye of the subject; detecting the brightness of at least two different areas in the sclera adjacent to the at least two different areas in the iris; in the respective adjacent areas, calculating ratios between the detected brightness of the at least two areas in the iris and the detected brightness of the at least two areas in the sclera; comparing the calculated brightness ratios between the different areas; and determining whether the subject is a living body or not according to the comparison results.

With this method, an eye image can be taken while the eye is irradiated with at least two illuminating rays in different radiation directions at different radiation angles to the eye of the subject at the same time. Then, in the at least two different areas in the iris of the taken eye image, ratios between the brightness of the iris and the brightness of the sclera are calculated and compared with each other. Thus, even one eye image taken allows determination of whether the image is of a living body or a forgery at a higher accuracy.

The present invention can provide a biometric discrimination system, an authentication system, and a biometric discrimination method that has fewer restrictions on the installation site and discriminate an unauthorized eye image using a forgery even when an unauthorized person attempts to pretend to be a registered person using a forgery. Thereby, the present invention can decrease the possibility of registration or authentication of unauthorized eye images.

### BRIEF DESCRIPTION OF THE DRAWINGS

Fig. 1 is an outline view showing an outline of an authentication system in accordance with a first exemplary embodiment of the present invention.
Fig. 2 is a schematic view illustrating the authentication system and a positional relation between illuminating light-emitting diodes (LEDs) and an eye of a subject in accordance with the first exemplary embodiment of the present invention.
Fig. 3A shows an appearance of the illuminating LEDs switched for light emission and eye images taken under the switched LEDs of the authentication system in accordance with the first exemplary embodiment of the present invention.
Fig. 3B shows an appearance of the illuminating LEDs switched for light emission and eye images taken under the switched LEDs of the authentication system in accordance with the first exemplary embodiment.
Fig. 3C shows an appearance of the illuminating LEDs switched for light emission and eye images taken under the switched LEDs of the authentication system in accordance with the first exemplary embodiment.
Fig. 3D shows an appearance of the illuminating LEDs switched for light emission and eye images taken under the switched LEDs of the authentication system in accordance with the first exemplary embodiment.
Fig. 3E shows an appearance of the illuminating LEDs switched for light emission and eye images taken under the switched LEDs of the authentication system in accordance with the first exemplary embodiment.
Fig. 4A is a graph showing iris contrasts calculated from eye images taken in the authentication system in accordance with the first exemplary embodiment.
Fig. 4B is a graph showing iris contrasts calculated from eye images taken in the authentication system in accordance with the first exemplary embodiment.
Fig. 5A is a schematic view illustrating illuminating rays from the authentication system in accordance with the first exemplary embodiment and a state in which the illuminating rays are reflected from a subject to be imaged.
Fig. 5B is a schematic view illustrating illuminating rays from the authentication system in accordance with the first exemplary embodiment and a state in which the illuminating rays are reflected from the subject to be imaged.
Fig. 5C is a schematic view illustrating illuminating rays from the authentication system in accordance with the first exemplary embodiment and a state in which the illuminating rays are reflected from the subject to be imaged.
Fig. 5D is a schematic view illustrating illuminating rays from the authentication system in accordance with the first exemplary embodiment and a state in which the illuminating rays are reflected from the subject to be imaged.
Fig. 6 is a block diagram showing a structure of an eye imaging apparatus and an iris authentication processor included in the authentication system in accordance with the first exemplary embodiment.
Fig. 7 is a flowchart showing the operation in the eye imaging apparatus and the iris authentication processor in accordance with the first exemplary embodiment.
Fig. 8 is a drawing showing areas in which brightness of an iris and brightness of a sclera is detected in a brightness detector of the authentication system in accordance with the first exemplary embodiment.
Fig. 9 is an outline view illustrating another example of the authentication system in accordance with the first exemplary embodiment.
Fig. 10 is an outline view illustrating yet another example of the authentication system in accordance with the first exemplary embodiment.
Fig. 11 is an outline view illustrating still another example of the authentication system in accordance with the first exemplary embodiment.
Fig. 12 is an outline view illustrating yet another example of the authentication system in accordance with the first exemplary embodiment.
Fig. 13 is a schematic view illustrating an authentication system and imaging positions of a subject in accordance with a second exemplary embodiment of the present invention.
Fig. 14 is a block diagram illustrating structures of an eye imaging apparatus and an iris authentication processor included in the authentication system in accordance with the second exemplary embodiment.
Fig. 15 is a schematic view illustrating an authentication system and imaging positions of a subject in accordance with a third exemplary embodiment of the present invention.
Fig. 16 is a block diagram illustrating structures of an eye imaging apparatus and an iris authentication processor provided in the authentication system in accordance with the third exemplary embodiment.
Fig. 17 is a schematic view illustrating an authentication system and an imaging position of a subject in accordance with a fourth exemplary embodiment of the present invention.
Fig. 18 is a block diagram illustrating structures of an eye imaging apparatus and an iris authentication processor included in the authentication system in accordance with the fourth exemplary embodiment.
Fig. 19 is a schematic view illustrating another example of the authentication system and an imaging position of a subject in accordance with the fourth exemplary embodiment.

### REFERENCE MARKS IN THE DRAWINGS

- 1, 100, 200, 300, 310: Authentication system
- 10, 101, 102: Illuminating unit
- 11: Illumination controller
- 12, 12a, 12b, 12c, 12d, 12e: Illuminating LED
- 13: Guide mirror
- 20, 201, 210: Imaging unit
- 21, 202, 211, 212: Lens
- 22, 221, 222: Image pickup device
- 23: Preprocessor
- 30, 301: Image signal processing unit
- 31: Pupil detector
- 32, 321: Brightness detector
- 33, 331: Determining part
- 34: Authentication image acquirer
- 40: Iris authentication processor
- 41: Register
- 50, 501, 502, 503: Eye imaging apparatus
- 60, 65: Instructing unit
- 61, 66: Instruction controller
- 62: Speaker
- 70a1, 70a2, 70b1, 70b2: Eye image
- 70c1, 70c2, 70d1, 70d2: Eye image
- 70e1, 70e2: Eye image
- 121: Movable part
- 203: Automatic-focusing controller
- 213: Switch

### DETAILED DESCRIPTION OF PREFERRED EMBODIMENTS

A biometric discrimination system of the present invention includes the following elements: an illuminating unit for irradiating an area including an eye of a subject with illuminating rays; an imaging unit for taking a plurality of images of the eye irradiated with the illuminating rays at different lighting angles to the eye of the subject; and a detector for detecting brightness of the iris in the taken eye images; and a determining part for comparing the detected brightness of the iris between the plurality of eye images and determining whether the subject is a living body or not according to the comparison results.

With this structure, a plurality of eye images are taken under illuminating rays at different lighting angles to an eye of a subject from the illuminating unit. Comparison of the brightness of the iris between the plurality of taken eye images allows the determination of whether the taken eye images are of a living body or a forgery.

Further, the detector may detect the brightness of the iris and the brightness of the sclera in the taken eye images. The determining part may calculate the ratios between the detected brightness of the iris and the detected brightness of the sclera and compare the calculated brightness ratios between the plurality of taken eye images. With this structure, in each of the plurality of taken eye images of the subject, the brightness of the iris and the brightness of the sclera are detected and the brightness ratios between the iris and the sclera are calculated. This structure can decrease operation errors that are caused by variations in the brightness of the illuminating rays to the eye of the subject, thus allowing accurate determination of whether the taken eye images are of a living body or a forgery.

Further, the biometric discrimination system may be structured to include a processor for processing the plurality of taken eye images so that the brightness of the sclera detected in the detector is constant in the plurality of taken eye images. With this structure, image processing allows the brightness of the sclera detected in the detector to be kept constant. Thereby, the brightness ratios between the iris and the sclera can be calculated at a higher accuracy. Because the brightness of the sclera can be set equal in the plurality of eye images, even only comparing the brightness of the iris between the plurality of taken eye images can determine whether the taken eye images are of a living body or a forgery.

Further, the biometric discrimination system may be structured to include a controller for controlling the brightness of the illuminating rays so that the brightness of the sclera detected in the detector is constant. With this structure, controlling the brightness of the illuminating rays allows the brightness of the sclera detected in the detector to be kept constant. Thus, the brightness ratios between the iris and the sclera can be calculated at a higher accuracy. Further, because the brightness of the sclera can be set equal in the plurality of eye images, even only comparing the brightness of the iris between the plurality of taken eye images can determine whether the taken eye images are of a living body or a forgery.

Further, the illuminating unit may be structured to have a plurality of light-emitting devices disposed at different distances from a specific point on the optical axis of the imaging unit and switched for light emission. Imaging the eye of the subject that are irradiated with illuminating rays at different lighting angles from the plurality of switched light-emitting devices provides a plurality of images of the eye irradiated with the illuminating rays at the different lighting angles to the eye of the subject. With this structure, at least two light-emitting devices disposed at different distances from one point on the optical axis of the imaging unit are switched to emit rays and illuminate the eye of the subject at at least two different lighting angles. Thus, this structure can provide the plurality of images of the eye of the subject irradiated with the illuminating rays at the different lighting angles to the eye of the subject. Now, the optical axis of the imaging unit means the optical axis of the lens included in the imaging unit.

Alternatively, the imaging unit may be structured to take a plurality of images of an eye of a subject irradiated with illuminating rays at different distances between the eye on the optical axis of the imaging unit and the imaging unit, thereby providing the plurality of images of the eye irradiated with the illuminating rays at the different lighting angles to the eye of the subject. With this structure, the distance between the eye on the optical axis of the imaging unit and the imaging unit is changed, and thereby a plurality of eye images of the subject under illuminating rays at different lighting angels can be provided.

Alternatively, the imaging unit may be structured to include at least two light receivers having optical axes parallel to each other so that imaging an eye of a subject on the respective optical axes of the at least two light receivers provide a plurality of images of the eye irradiated with illuminating rays at different lighting angles to the eye. With this structure, imaging the eye of the subject on the respective parallel optical axes of the at least two light receivers can provide a plurality of images of the eye irradiated with illuminating rays at different lighting angles to the eye.

The biometric discrimination system may be structured to include an instructing unit for instructing the subject to change the imaging position. With this structure, the subject can change the imaging position according to the instruction from the instructing unit, and thus changing the imaging position makes taking eye images easier.

A biometric discrimination system of the present invention may include the following elements: an illuminating unit for irradiating an eye of a subject with at least two illuminating rays in different radiation directions at different lighting angles; an imaging unit for imaging the eye of the subject irradiated with the illuminating rays; a detector for detecting the brightness of at least two different areas in the iris in a taken eye image that are set according to the radiation directions of the illuminating rays to the eye; and a determining part for comparing the detected brightness of the iris between the at least two different areas. Whether the subject is a living body or not may be determined by the comparison results of the determining part.

This structure can provide an image of the eye of the subject irradiated with at least two illuminating rays in different radiation directions at different lighting angles to the eye at the same time. Thus, even one eye image taken allows determination of whether the eye image is of a living body or a forgery.

Further, the detector may detect the brightness of at least two different areas in the sclera set adjacent to the at least two different areas in the iris. The determining part may calculate the ratios between the brightness of the iris detected in the at least two different areas and the brightness of the sclera detected in the at least two different areas in the respective adjacent areas, and compare the calculated brightness ratios between the different areas. With this structure, the brightness ratios between the iris and the sclera are calculated in the adjacent areas in the taken eye image, and the calculated brightness ratios are compared between the different areas. This structure can decrease operation errors caused by variations in the brightness of the illuminating rays to the eye of the subject, thus allowing accurate determination of whether the taken eye image is of a living body or a forgery.

Further, the illuminating unit may be structured to irradiate the eye of the subject disposed on the optical axis of the imaging unit with illuminating rays from the outer side and the inner side of the eye at different lighting angles. In this structure, because the eye of the subject is irradiated with the illuminating rays from the outer side and the inner side of the eye disposed on the axis of the imaging unit, the areas for detecting the brightness of the taken eye image can be set in two positions in the iris on the outer side and the inner side of the eye. Thus, the brightness of the iris and the sclera can be detected in the taken eye image at a higher accuracy.

Further, the biometric discrimination system may be structured to include a controller. The detector detects the brightness of at least two different areas in the sclera set adjacent to at least two different areas in the iris. Then, the controller controls the brightness of the illuminating rays so that the brightness of the at least two different areas in the sclera detected in the detector is kept constant. With this structure, because controlling the brightness of the illuminating rays can make the brightness of the sclera detected in the detector equal in different areas, the brightness ratios between the iris and the sclera can be calculated at a higher accuracy. Further, this structure can make the brightness of the different areas in the sclera equal, and thus even only comparing the brightness between the at least two different areas in the iris can determine whether the taken eye image is of a living body or a forgery.

Further, the illuminating unit may be structured to radiate near infrared rays. Because irises have characteristics of easily radiating near infrared rays, this structure can provide a more vivid eye image and determine whether the taken eye image is of a living body or a forgery at a higher accuracy.

Further, an authentication system of the present invention includes the biometric discrimination system and an authentication processor for performing authenticating operation using the iris portion of an eye image determined not to be of a forgery in the biometric discrimination system. In this structure, whether the taken eye image is of a living body or a forgery is determined first, and iris authentication operation is performed only on the eye image determined not to be of a forgery. Thus, this structure can decrease the possibility of authentication of an unauthorized, pretending person.

Further, the biometric discrimination system may be structured to have a register for registering information on the iris of the eye image determined not to be of a forgery. This structure allows the determination of whether the taken eye image is of a living body or a forgery and the registration of the information on the iris of the eye image determined not to be of a forgery. Thereby, this structure decreases the possibility of registration of an unauthorized, pretending person.

A biometric discrimination method of the present invention includes: changing the lighting angle of illuminating rays from a illuminating unit to an eye of a subject; imaging the eye of the subject irradiated with the illuminating rays at different lighting angles; detecting brightness of the iris and brightness of the sclera of the eye images taken; calculating ratios between the detected brightness of the iris and the detected brightness of the sclera; comparing the calculated brightness ratios between the plurality of eye images; and determining whether or not the subject is of a living body, according to the comparison results.

With this method, the plurality of eye images are taken under illuminating rays at different lighting angles to the eye of the subject, and comparison of the brightness ratios between the iris and the sclera in the plurality of taken eye images of the subject can determine whether the taken eye images are of a living body or a forgery.

A method of discriminating a living body using the biometric discrimination system of the present invention includes: irradiating an eye of a subject with at least two illuminating rays in different radiation directions at different lighting angles at the same time; imaging the eye of the subject irradiated with the illuminating rays; in the taken eye image, detecting the brightness of at least two different areas in the iris that are set according to the radiation directions of the illuminating rays to the eye of the subject; detecting the brightness of at least two different areas in the sclera set adjacent to the at least two different areas in the iris; in the respective adjacent areas, calculating the ratios between the detected brightness of the at least two areas in the iris and the detected brightness of the at least two areas in the sclera; comparing the calculated brightness ratios between the different areas; and determining whether the subject is a living body or not according to the comparison results.

With this method, an eye image can be taken while the eye is irradiated with at least two illuminating rays in different radiation directions at different radiation angles to the eye of the subject at the same time. Then, in the at least two different areas in the iris in the taken eye image, the brightness ratios between the iris and the sclera are calculated and compared with each other. Thus, even one eye image taken allows determination of whether the image is of a living body or a forgery at a higher accuracy.

Hereinafter, descriptions are provided of exemplary embodiments of the present invention with reference to the accompanying drawings.

### FIRST EXEMPLARY EMBODIMENT

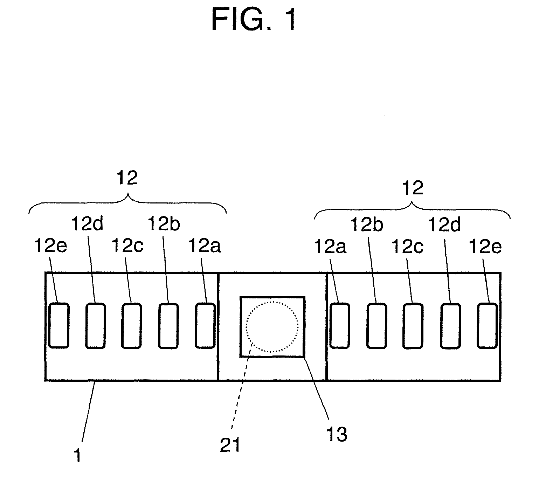

A description is provided of an authentication system of the first exemplary embodiment of the present invention, with reference to Fig. 1. Fig. 1 is an outline view illustrating an outline of authentication system 1 of the first exemplary embodiment.

Authentication system 1 includes the following elements: guide mirror 13 to be used when a subject checks the position of the eye with an image reflected therefrom in imaging the eye of the subject; illuminating LED parts 12 made of known light-emitting devices for emitting near infrared rays; and lens 21 disposed behind guide mirror 13. Illuminating LED parts 12 include five pairs of illuminating LEDs 12a, 12b, 12c, 12d, and 12e disposed symmetrically about the optical axis of lens 21. Each of the five pairs can be switched to emit rays separately. Authentication system 1 takes images including the eye of the subject, i.e. eye images, under illuminating rays emitted from switched illuminating LEDs 12. Thus, in authentication system 1, a plurality of eye images are taken under the illuminating rays from illuminating LED parts 12 at different angles to the eye of the subject. Authentication system 1 also determines whether or not the plurality of taken eye images are of a forgery. For the eye images determined not to be of a forgery, the authentication system performs iris authentication operation using the iris portion of the taken eye images to determine if the subject is an already registered person, and supplies the result as an electrical signal.

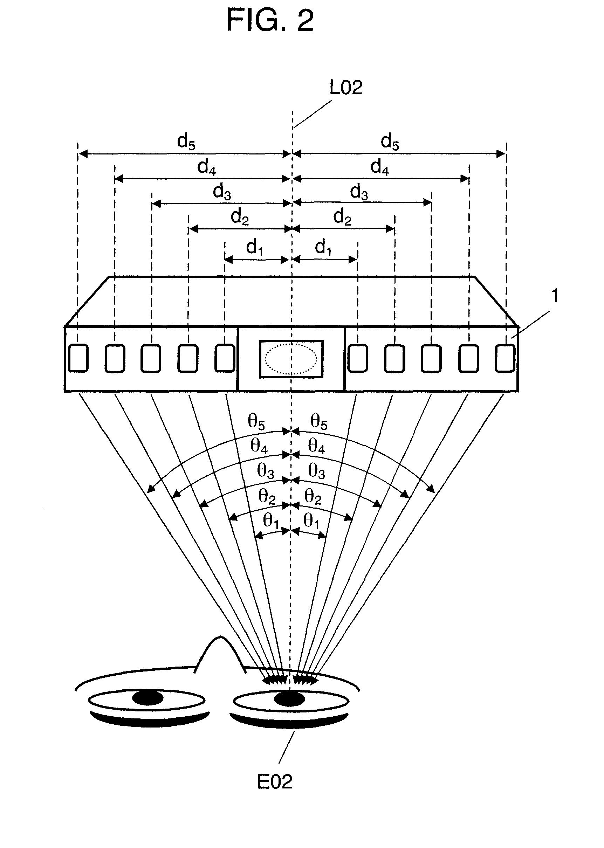

Fig. 2 is a schematic view illustrating authentication system 1 and a positional relation between illuminating LEDs 12 and an eye of a subject in the first exemplary embodiment of the present invention. Illuminating LEDs 12 are used as the generic term of illuminating LEDs 12a, 12b, 12c, 12d, and 12e.

As shown in Fig. 2, five pairs of illuminating LEDs 12a, 12b, 12c, 12d, and 12e symmetrical about optical axis L02 of lens 21 are disposed at distances of d1, d2, d3, d4, and d5 from the center of lens 21, respectively.

The angles of illuminating rays from illuminating LEDs 12a, 12b, 12c, 12d, and 12e to an eye of subject E02 disposed on the optical axis of lens 21 (hereinafter referred to as "lighting angle") are θ1, θ2, θ3, θ4, and θ5, respectively. Thus, the lighting angle of illuminating LED 12a disposed nearest to lens 21 is the smallest angle of θ1. The lighting angle of illuminating LED 12e disposed furthest to the center of lens 21 is the largest angle of θ5. In this exemplary embodiment, illuminating LEDs 12a, 12b, 12c, 12d, and 12e are disposed so that θ1 is 10°, θ2 is 20°, θ3 is 30°, θ4 is 40°, and θ5 is 50°.

In the first exemplary embodiment of the present invention, illuminating LEDs 12a, 12b, 12c, 12d, and 12e are switched for light emission, a plurality of eye images of the subject are taken while the eye is irradiated with illuminating rays at different lighting angles to the eye, and the taken eye images are determined to be of a forgery or a living body.

The inventors have found that the difference in brightness between the iris and the sclera ("iris contrast") varies from a living body to a forgery, when eye images are taken under illuminating rays at different lighting angles. Authentication system 1 of the first exemplary embodiment of the present invention uses this phenomenon to discriminate a living body. Taking a plurality of eye images under illuminating rays at different lighting angles, detecting the iris contrast in each of the taken eye images, and comparing the detected iris contrasts between the plurality of taken eye images can determine whether the eye images are of a forgery or a living body and prevent false pretense.

Now, a description is provided of an experiment conducted by the inventors, i.e. a phenomenon of the difference in the brightness of the iris portion between a living body and a forgery when eye images are taken under illuminating rays at different lighting angles to an eye of a subject.

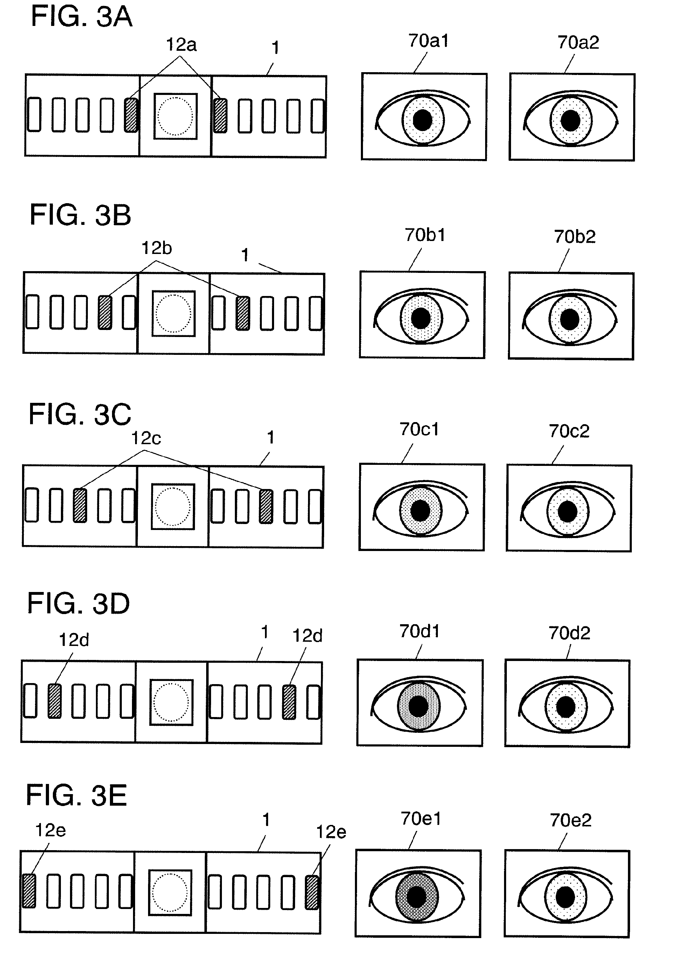

Fig. 3 shows appearances of illuminating LEDs 12 of authentication system 1 switched for light emission, and eye images taken under the switched LEDs in the first exemplary embodiment. Now, Figs. 3A through 3E are generically called Fig. 3. In Fig. 3, illuminating LEDs 12 emitting rays are hatched. In Fig. 3, eye images 70a1 through 70e1 are of a living eye, and eye images 70a2 through 70e2 are of the eye wearing a contact lens with an iris pattern printed thereon (hereinafter referred to as "artificial eye contact lens"). In respective eye images of Fig. 3, the sclera portions are of a living body and in common, and only iris portions are different in that they are of a living eye or an artificial contact lens.

Fig. 3A shows eye images taken when illuminating LEDs 12a emit rays to irradiate an eye of a subject with illuminating rays at the smallest lighting angle of θ1 to the eye. Fig. 3B shows eye images taken when illuminating LEDs 12b emit rays to irradiate the eye of the subject with illuminating rays at a lighting angle of θ2 to the eye. Fig. 3C shows eye images taken when illuminating LEDs 12c emit rays to irradiate the eye of the subject with illuminating rays at a lighting angle of θ3 to the eye. Fig. 3D shows eye images taken when illuminating LEDs 12d emit rays to irradiate the subject with illuminating rays at a lighting angle θ4 to the eye. Fig. 3E shows eye images taken when illuminating LEDs 12e emit rays to irradiate the eye of the subject with illuminating rays at the largest lighting angle of θ5 to the eye. In Fig. 3, θ1 is 10°, θ2 is 20°, θ3 is 30°, θ4 is 40°, and θ5 is 50°.

As shown in Figs. 3A and 3B, there is no large difference in the brightness of the iris between eye images 70a1 and 70b1 of a living eye and eye images 70a2 and 70b2 of the eye wearing an artificial contact lens taken under illuminating rays from illuminating LEDs 12 at small lighting angles. However, as shown in Figs. 3C and 3D, at larger lighting angles of illuminating rays from illuminating LEDs 12, the brightness of the iris in eye images 70c1 and 70d1 of the living eye is smaller and has clear difference from the brightness of the iris in eye images 70c2 and 70d2 of the eye wearing the artificial contact lens. Then, as shown in Fig. 3E, at the largest lighting angle of illuminating rays from illuminating LEDs 12, the iris in eye image 70e1 of the living eye is the darkest and has a large difference in brightness from the iris of eye image 70e2 of the eye wearing the artificial contact lens.

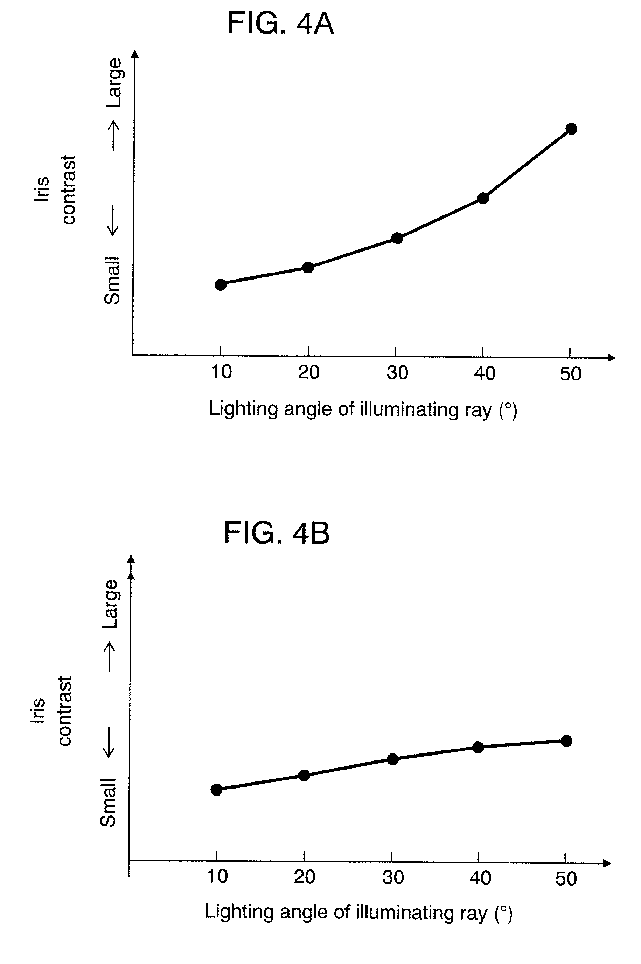

Figs. 4A and 4B are graphs showing iris contrasts calculated from eye images taken in authentication system 1 of the first exemplary embodiment of the present invention. Fig. 4A is a graph showing iris contrasts calculated from eye images of a living eye. Fig. 4B is a graph showing iris contrasts calculated from eye images of the eye wearing an artificial contact lens. Now, in this exemplary embodiment, the brightness of specific areas in the sclera is detected as "the brightness of the sclera", and the brightness of specific areas in the iris is detected as "the brightness of the iris". A value obtained by dividing the brightness detected in the sclera by the brightness detected in the iris is used as "iris contrast". This is because calculating iris contrasts in the form of ratios resulting from division can decrease errors, such as variations in the luminance or light quantity of the illuminating rays in the plurality of taken eye images, in comparison with simple calculation of the brightness of the iris. In Fig. 4, the X axis represents lighting angles (°). A larger value indicates a larger lighting angle of illuminating rays to the eye of the subject. The Y axis represents iris contrasts. A larger value indicates a larger iris contrast, that is, a larger difference in brightness between the sclera and the iris.

As shown in Fig. 4A, for eye images of a living eye, the iris contrast is the smallest at a lighting angle of 10° of illuminating rays from illuminating LEDs 12. The iris contrast is larger at a larger lighting angle, and is the largest at a lighting angle of 50°. On the other hand, for eye images of the eye wearing an artificial contact lens, as shown in Fig. 4B, the iris contrast values at lighting angles 10° and 20° have no significant difference from the iris contrast values of Fig. 4A. For the eye wearing the artificial contact lens, the iris contrast does not change significantly at larger lighting angles. As a result, at a lighting angle of 50°, the iris contrast value of Fig. 4B has a large difference from the iris contrast value of Fig. 4A.

The reason for such a phenomenon is inferred as follows.

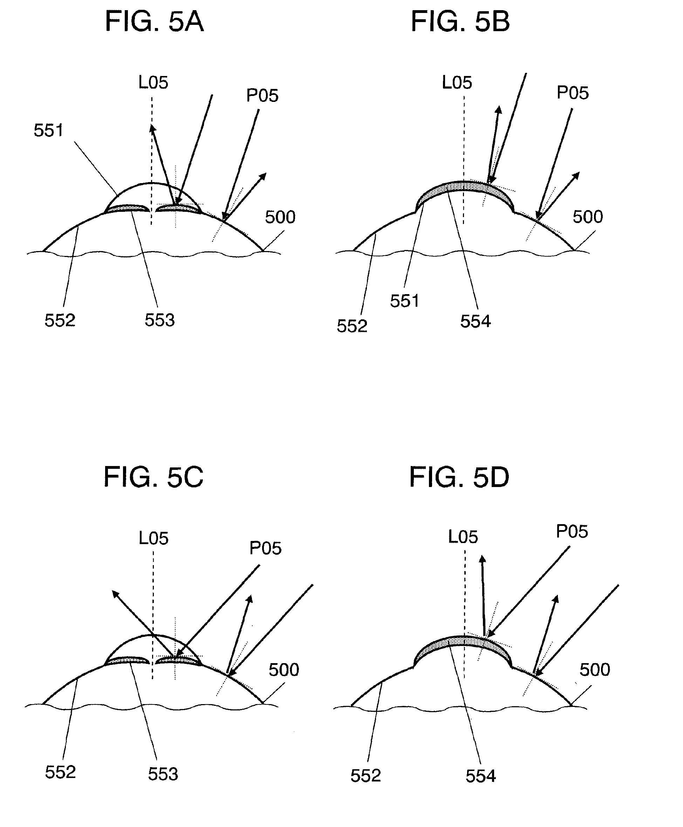

Fig. 5 shows schematic views of illuminating rays from authentication system 1 of the first exemplary embodiment and states in which the illuminating rays are reflected from subjects to be imaged. Figs. 5A, 5B, 5C, and 5D are generically called as Fig. 5.

In each of Figs. 5A and 5C, the subject to be imaged is a living eye. Eye ball 500 is covered with cornea 551 and sclera 552, and has iris 553 inside of cornea 551. On the other hand, in each of Figs. 5B and 5D, the subject to be imaged is the eye that wears an artificial contact lens having an iris pattern printed thereon. In other words, cornea 551 is covered with artificial contact lens 554 having a forged iris formed thereon. Figs. 5A and 5B show cases of a small lighting angle of illuminating rays L05 to the eye of the subject. Figs. 5C and 5D show cases of a large lighting angle of illuminating rays L05.

As shown in Fig. 5, iris 553 in the living eye and artificial contact lens 554 having an iris pattern printed thereon are different in position and shape. In other words, in the living eye, iris 553 is behind cornea 551 and has a shape flatter than the surface of cornea 551. On the other hand, in the eye wearing artificial contact lens 554, artificial contact lens 554 is on the surface of cornea 551 and has a shape bent like the surface of cornea 551.

As shown in Figs. 5A and 5B, illuminating rays P05 having a smaller lighting angle are reflected at small angles to optical axis L05 of lens 21 in the portions of iris 553 and sclera 552 for both of the living eye and the eye wearing the artificial contact lens. Thus, the brightness of iris 553 and the brightness of sclera 552 detected in the taken eye images are high for both of the living eye and the eye wearing the artificial contact lens. The difference in brightness between the iris and the sclera is small for both eyes, and thus the calculated iris contrasts are small to the same degree.

Next, a description is provided of cases of illuminating rays P05 at a larger lighting angle, with reference to Figs. 5C and 5D. For the living eye of Fig. 5C, because the portion of iris 553 is flatter than the surface of the eye ball, illuminating ray P05 having a larger lighting angle is reflected on the surface of iris 553 at a larger angle to optical axis L05 of lens 21. On the other hand, for the eye wearing artificial contact lens 554 of Fig. 5D, artificial lens 554 is on the surface of eye ball 500 and bent like the surface of the eye ball. For this reason, illuminating ray P05 is reflected on the surface of artificial contact lens 554 at a smaller angle to optical axis L05 of lens 21 than the illuminating ray in the living eye. In other words, illuminating ray P05 is reflected in the direction of lens 21. Consequently, the iris portion of the living eye has lower brightness than the eye wearing the artificial contact lens in the taken eye images.

As described above, the iris contrasts detected in the taken eye images have no significant difference under illuminating rays at smaller lighting angles between a living eye and the eye wearing an artificial contact lens. However, when the illuminating ray has a larger lighting angle, the lighting angle to the iris portion in a living eye is larger than that in the eye wearing an artificial contact lens. As a result, for the living eye, the taken eye image has a dark iris portion, and the calculated iris contrast value is larger. In contrast, for the eye wearing an artificial contact lens, the taken eye image has a bright iris portion even under illuminating rays having a large lighting angle, and the calculated iris contrast value remains small.

Thus, taking at least two eye images under illuminating rays at different lighting angles, detecting iris contrasts in each of the eye images, and comparing the detected iris contrasts with each other can determine whether the taken eye images are of a living body or a forgery.

In other words, for the first exemplary embodiment of the present invention, taking a plurality of eye images under illuminating rays from illuminating LEDs 12 at different lighting angels to the eye of the subject, detecting the brightness of the iris and the brightness of the sclera in each of the eye images for calculation of iris contrasts, and comparing the calculated iris contrasts with each other can determine whether the taken eye images are of a living eye or a forgery and prevent false pretense.

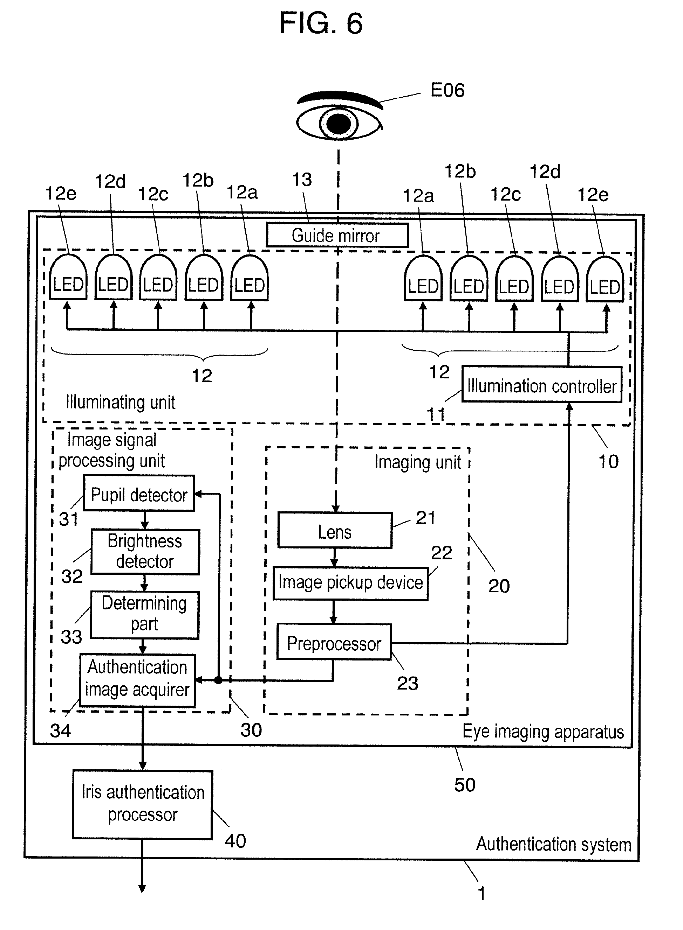

Fig. 6 is a block diagram illustrating structures of eye imaging apparatus 50 and iris authentication processor 40 included in authentication system 1 of the first exemplary embodiment of the present invention. As shown in Fig. 6, authentication system 1 includes eye imaging apparatus 50 for taking images of eye E06 of a subject and iris authentication processor 40 for authenticating the iris using the eye images taken by eye imaging apparatus 50.

Eye imaging apparatus 50 includes guide mirror 13, illuminating unit 10 for illuminating the subject, imaging unit 20 for taking eye images, and image signal processing unit 30 for processing the signals of the eye images taken by imaging unit 20.

Guide mirror 13 disposed in front of lens 21 is used when the subject checks the position of the eye of the subject by looking at an image reflected therefrom. Guide mirror 13 is made of a commonly known semi-transmissive material, and reflects and transmits light at the same time. A part of the transmitted light is fed into imaging unit 20.

Illuminating unit 10 includes illuminating LED parts 12 made of known light-emitting devices for emitting near infrared rays, such as a light-emitting diode, and illumination controller 11 for controlling illumination of illuminating LEDs 12. As described above, illuminating LED parts 12 are made of five pairs of illuminating LEDs 12a, 12b, 12c, 12d, and 12e that are disposed symmetrically about the optical axis of lens 21 at different distances from the center of lens 21 and illuminate the area including the eye of the subject. The illuminating rays that are emitted by illuminating LEDs 12a disposed at a smaller distance from lens 21 illuminate the eye of the subject at a smaller lighting angle. The illuminating rays that are emitted by illuminating LEDs 12e disposed at a larger distance from the center of lens 21 illuminate the eye of the subject at a larger lighting angle. Illumination controller 11 switches these five pairs of illuminating LEDs 12a, 12b, 12c, 12d, and 12e for light emission, and controls the emission luminance so that a light quantity suitable for eye image acquisition can be obtained.

Imaging unit 20 includes lens 21 made of a commonly used fixed-focus lens, image pickup device 22 made of a known element, such as a charge-coupled device (CCD), and preprocessor 23. Near infrared rays emitted from illuminating LEDs 12 are reflected from the eye of the subject and surrounding areas thereof, and the reflected light is fed into image pickup device 22 via lens 21. The incident light in image pickup device 22 is subjected to photoelectric conversion therein, and fed into preprocessor 23 as electrical signals. Preprocessor 23 extracts image signal component from the electrical signals supplied from image pickup device 22, and determines the image quality relating to contrast, focusing, or the like. Next, preprocessor 23 performs processing operation necessary for image signals, e.g. gain adjustment, and supplies the processed signals to image signal processing unit 30. Preferably, lens 21 and image pickup device 22 may be structured to include filters having characteristics of transmitting near infrared rays and cutting visible light.

Image signal processing unit 30 includes the following elements: pupil detector 31 for detecting the position of the pupil from the eye images taken in imaging unit 20; brightness detector 32 for detecting the brightness of the iris and the brightness of the sclera from the taken eye images; determining part 33 for determining whether the taken eye images are of a forgery or a living eye from the brightness of the iris and the brightness of the sclera detected in brightness detector 32; and authentication image acquirer 34 for acquiring the eye images taken in imaging unit 20 as eye images for authentication, according to the results of determining part 33.

Pupil detector 31 detects the position of the pupil in the signals of the eye images supplied from preprocessor 23. The methods of detecting the pupil position in the signals of the eye images include commonly known techniques, such as using template matching and circumference integration (see Japanese Patent Unexamined Publication No. H08-504979, for example).

Brightness detector 32 determines areas in the iris and areas in the sclera for detecting the brightness thereof according to the position of the pupil detected in pupil detector 31, and detects the average brightness of the determined iris areas and the average brightness of the determined sclera areas as iris brightness and sclera brightness, respectively. The areas in the iris and the areas in the sclera for detecting the average brightness are described later.

Determining part 33 divides the brightness of the sclera by the brightness of the iris both detected in brightness detector 32, and provides the calculated result as an iris contrast. Then, the determining part calculates the iris contrast for each of the plurality of taken eye images, compares the calculated iris contrasts with each other, and determines whether the taken eye images are of a forgery or a living eye. Thereafter, determining part 33 outputs signals representing the determination results to authentication image acquirer 34. An example of the determining method in determining part 33 includes: obtaining a difference in iris contrast between an eye image taken under illuminating rays at the smallest lighting angle and another eye image taken at the largest lighting angle; comparing the difference with a threshold; and determining the image having a difference smaller than the threshold is of a forgery and the image having a difference larger than the threshold is of a living eye. In this manner, determining part 33 can determine whether the taken eye images are of a living eye or a forgery. Preferably, the threshold is predetermined according to experiments or the like.

Authentication image acquirer 34 receives signals from determining part 33. When the signals indicate that the taken eye images are not of a forgery, the authentication image acquirer acquires signals of the eye images supplied from preprocessor 23 and outputs the signals to iris authentication processor 40 as eye images for authentication.

Iris authentication processor 40 extracts an image of the iris area from the eye images for authentication supplied from image signal processing unit 30, and creates authentication information based on the wrinkle patterns of the iris portion. Then, the iris authentication processor compares the authentication information with already registered authentication information, and determines whether or not both pieces of information correspond with each other and the subject is a registered person. The function of iris authentication processor 40 can be implemented by such a known method as disclosed in Japanese Patent Unexamined Publication No. 2000-33080.

In this manner, in eye imaging apparatus 50 and iris authentication processor 40 of the first exemplary embodiment of the present invention, a plurality of eye images are taken under illuminating rays at different lighting angels to an eye of a subject, and the brightness of the iris and the brightness of the sclera are detected from each of the taken eye images for calculation of iris contrasts. Then, the eye imaging apparatus compares the calculated iris contrasts with each other and determines whether the taken eye images are of a living eye or a forgery. When the taken eye images are determined not to be of a forgery, the iris authentication processor compares the iris patterns of the taken eye images and performs authenticating operation to determine whether the subject is an already registered person.

The function of each of image signal processing unit 30, preprocessor 23, illumination controller 11, and iris authentication processor 40 may be implemented by hardware. Alternatively, each of the functions may be described to be implementable by software and implemented by an arithmetic unit or the like. When the function is implemented by software, each of eye imaging apparatus 50 and iris authentication processor 40 can be structured of a computer that includes an arithmetic unit having loaded thereon programs for executing the function block.

The major elements for discriminating a living body in the first exemplary embodiment are illuminating unit 10, imaging unit 20, brightness detector 32, and determining part 33. A system including at least these elements is called a biometric discrimination system. The biometric discrimination system of the first exemplary embodiment may include the entire part of image signal processing unit 30.

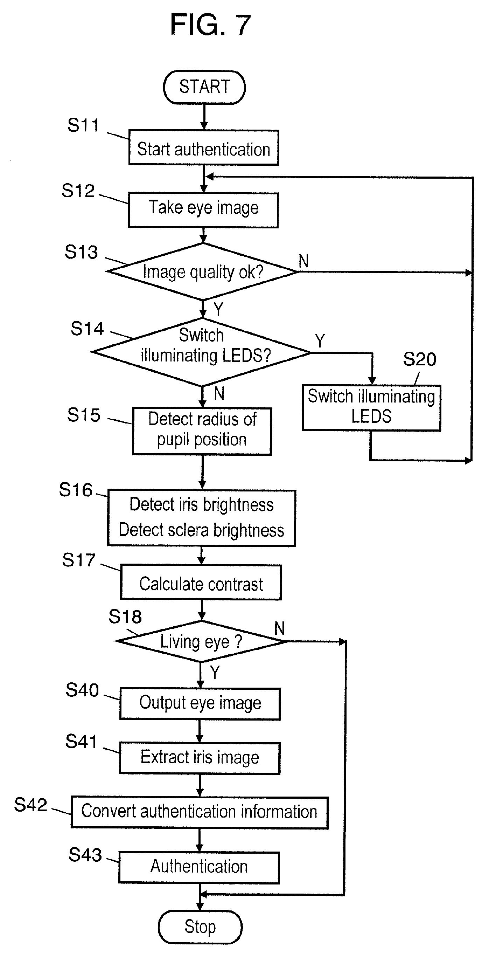

Fig. 7 is a flowchart that shows the operation of eye imaging apparatus 50 and iris authentication processor 40 of the first exemplary embodiment. First, a subject places the face in front of authentication system 1, and properly positions the eye while looking at guide mirror 13. Thereafter, inputting instructions to start authentication or the like causes the system to start authentication operation (S11).

Then, illumination controller 11 causes illuminating LED12a disposed in the nearest position to lens 21 to emit rays, and imaging unit 20 takes an eye image of the subject irradiated with illuminating rays from illuminating LED12a (S12).

Preprocessor 23 determines if the image quality relating to focusing, luminance, contrast, or the like of the acquired eye image is appropriate. When the image quality is not appropriate, necessary processing, such as instructions to the subject, is performed again, and another eye image is acquired (S13).

Illumination controller 11 determines if illuminating LEDs emitting rays are illuminating LEDs 12e in the furthest position from lens 21 (S14). When the ray is no from illuminating LEDs 12e, the illuminating LEDs outside of those emitting rays at that time are switched to emit rays (S20). Then, the flow returns to S12, and another image is acquired.

In step S14, when illuminating LEDs 12 emitting rays are determined to be illuminating LEDs 12e in the furthest position from lens 21, pupil detector 31 detects the position and radius of the pupil from each of the plurality of taken eye images (S15).

Then, brightness detector 32 determines the iris position and the sclera position in each of the plurality of eye images according to the detected position and radius of the pupil. Then, the brightness detector determines the areas for detecting the brightness of the iris and the areas for detecting the brightness of the sclera from the determined iris position and sclera position, and detects the brightness of these areas by, for example, obtaining the average luminance in the areas (S16).

Determining part 33 divides the brightness detected in the sclera by the brightness detected in the iris, to provide iris contrasts (S17).

Determining part 33 compares the values of iris contrast calculated in the plurality of eye images taken under illuminating rays at different lighting angels to the eye of the subject. For example, comparing the difference in iris contrast between an eye image taken at the smallest lighting angle and another eye image taken at the largest lighting angle with a threshold determines whether the taken eye images are of a forgery or a living eye (S18).

Authentication image acquirer 34 supplies the eye images determined to be of a living eye by determining part 33, to iris authentication processor 40, as eye images for authentication (S40).

Iris authentication processor 40 extracts an iris image from the eye image data, according to the coordinates of the center of the pupil (S41). Then, the iris image is converted into specific authentication information numerically expressing the iris pattern (S42). The authentication information is compared with registered authentication information for authentication operation (S43).

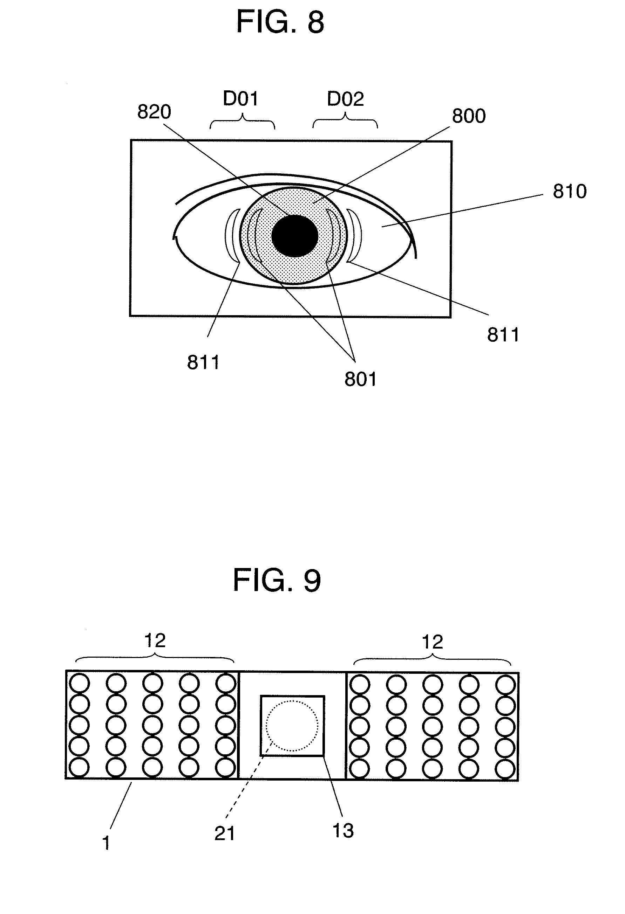

Next, a description is provided of areas in which the brightness of the iris and the brightness of the sclera are detected in the eye images. Fig. 8 shows the areas in which brightness detector 32 of authentication system 1 of the first exemplary embodiment detects the brightness of the iris and the brightness of the sclera in the eye images.

In the first exemplary embodiment of the present invention, the areas for detecting the brightness of the iris and the brightness of the sclera are determined according to the position and radius of pupil 820 detected in pupil detector 31, and radiation directions of illuminating rays from illuminating LEDs12 to the eye of the authenticatee.

Fig. 8 shows areas in iris 800 and areas in sclera 810 in which the brightness is detected. Provided in two positions near the boundary between iris 800 and sclera 810, i.e. area D01 on the outer side of the eye and area D02 on the inner side of the eye, are substantially rectangular or crescent detection areas 801 and 811 each having a major axis along the boundary of iris 800 and sclera 810. Areas 801 for detecting the brightness of the iris and areas 811 for detecting the brightness of the sclera are provided in the two positions because illuminating LEDs 12 illuminate the eye of the subject in two directions, i.e. from the outer side and the inner side of the eye. The areas for detecting the brightness are set as substantially rectangular or crescent shapes near the boundary between iris 800 and sclera 810 because experiments show that detecting areas having iris 800 and sclera 810 adjacent to each other can provide a clearer brightness difference than detecting areas further from the boundary between iris 800 and sclera 810.

As described above, authentication system 1 of the first exemplary embodiment of the present invention takes a plurality of eye images under illuminating rays at different lighting angles to an eye of a subject. Then, the brightness of the iris and the brightness of the sclera are detected from the taken eye images to provide iris contrasts, and the calculated iris contrasts are compared with each other to determine whether or not the taken eye images are of a living eye or a forgery. This system can prevent false pretense of an unauthorized person using a forgery or the like.

In the description of the first exemplary embodiment of the present invention, illuminating LED parts 12 are made of five pairs of illuminating LEDs 12a, 12b, 12c, 12d, and 12e in one row so that each pair is disposed on the right and left sides. However, the present invention is not limited to this structure. Fig. 9 is an outline view illustrating another example of authentication system 1 of the first exemplary embodiment. For example, as shown in Fig. 9, each of illuminating LED parts 12 may be structured of five rows of LEDs. When illuminating LED parts 12 are made of a plurality of rows of LEDs in this manner, each LED may have a relatively low emission luminance. Changing the number of LEDs emitting rays can control the light quantity during illumination of the eye of the subject.

In the description of the first exemplary embodiment, illuminating LED parts 12 are made of five pairs of illuminating LEDs 12a through 12e. In this structure, "false pretense" is prevented by increasing the number of combinations of lighting angles of the illuminating rays to the eye of the subject when a plurality of eye images are taken. However, this exemplary embodiment is not limited to this structure. For example, illuminating LED parts 12 may be structured of five or more, or five or fewer pairs of LEDs.

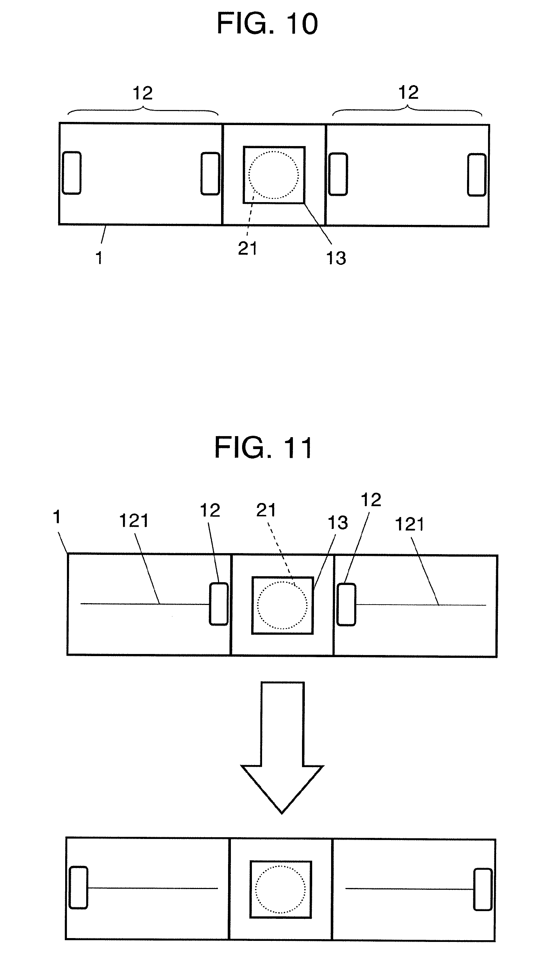

Fig. 10 is an outline view of another example of authentication system 1 of the first exemplary embodiment of the present invention. As shown in Fig. 10, illuminating LED parts 12 may be made of two pairs of LEDs. In the first exemplary embodiment, taking at least two eye images under illuminating rays at different angles to an eye of a subject can provide the above advantage. Therefore, in the first exemplary embodiment, taken eye images can be determined to be of a living eye or a forgery with at least two pairs of illuminating LEDs.

Described in the first exemplary embodiment of the present invention is a structure in which five pairs of illuminating LEDs 12a through 12e in illuminating LED parts 12 disposed on the right and left sides are switched for light emission. However, the present invention is not limited to this structure. Fig. 11 is an outline view of another example of authentication system 1 of the first exemplary embodiment. As shown in Fig. 11, an authentication system may include only a pair of illuminating LEDs 12 and movable parts 121 capable of moving the illuminating LEDs. In this structure, the pair of illuminating LEDs 12 can be moved to the closest position to lens 21 (the upper drawing in Fig. 11) and the furthest position thereto (the lower drawing in Fig. 11) by movable parts 121. The movement of the LEDs in this manner allows at least two eye images to be taken under illuminating rays at different lighting angles to an eye of a subject. The lighting angles of illuminating rays to the eye of the subject may be changed by physically moving illuminating LEDs 12. The methods of changing the lighting angles include changing the optical paths of the illuminating rays from illuminating LEDs 12 by refraction and reflection of light, using commonly known means of reflection, refraction, and spectroscopy, such as a lens, prism, and mirror, and combinations thereof.

In the first exemplary embodiment of the present invention, the areas for detecting the brightness of the iris and the brightness of the sclera are set on the outer side and the inner side of the eye near the boundary between the iris and the sclera. However, when the eye of the subject is irradiated with illuminating LEDs 12 in directions other than the outer side and the inner side of the eye, the areas for detecting the brightness of the iris and the brightness of the sclera are set according to the radiation directions.

Described in the first exemplary embodiment is a structure in which the areas for detecting the brightness of the iris are set in two positions and the areas for detecting the brightness of the sclera are set in two positions. For example, an area along the boundary between the iris and sclera may be set on each of the outer side and inner side so as to form a ring shape. In the first exemplary embodiment, the brightness of the iris and the brightness of the sclera are obtained by calculating the average luminance in the areas for detecting the brightness. However, any method of calculating the brightness in numerical values including commonly known brightness detection methods can be used. In the first exemplary embodiment, the iris contrasts are calculated on each of the outer side and inner side of the eye near the boundary between the iris and the sclera in the taken images, and the average value of the calculated iris contrasts is provided as the iris contrast of the taken eye images. However, the iris contrast may be calculated as a result of obtaining the brightness of the iris both on the outer side and the inner side of the eye as the brightness of the iris, and obtaining the brightness of the sclera both on the outer side and the inner side of the eye as the brightness of the sclera.

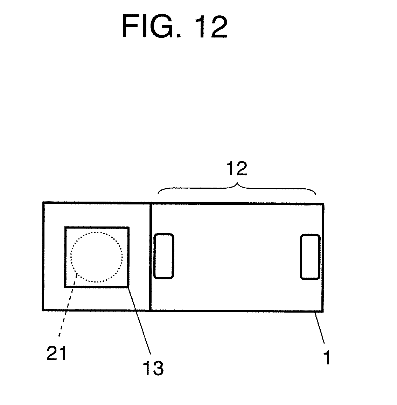

Described in the first exemplary embodiment is a structure including pairs of illuminating LEDs 12 disposed symmetrically about the optical axis of lens 21. However, the present invention is not limited to this structure. Fig. 12 is an outline view illustrating yet another example of authentication system 1 of the first exemplary embodiment of the present invention. As shown in Fig. 12, illuminating LED part 12 may be structured of at least two illuminating LEDs disposed at different distances from lens 21 on one of the right and left sides of lens 21. This structure can provide an advantage similar to that described above. However, in this structure, the areas for detecting the brightness of the iris and the brightness of the sclera are not in the two positions on each of the outer side and the inner side of the eye near the boundary between the iris and sclera as described with reference to Fig. 8. Preferably, when the eye is irradiated with the illuminating LED from the outer side of the eye, areas near the boundary between the iris and sclera on the outer side of the eye, i.e. on the side irradiated with the illuminating LEDs, are detected.

### SECOND EXEMPLARY EMBODIMENT

Next, a description is provided of an authentication system of the second exemplary embodiment of the present invention, with reference to the accompanying drawings.

Described in the first exemplary embodiment is a structure in which illuminating LEDs 12 are made of a plurality of illuminating LEDs 12a, 12b, 12c, 12d, and 12e, and taking eye images under these LEDs switched for light emission provides a plurality of eye images under illuminating rays at different lighting angles to an eye of a subject. However, the plurality of eye images can be taken under illuminating rays at different lighting angles to the eye of the subject not under switched illuminating LEDs 12a, 12b, 12c, 12d, and 12e for light emission but at the distance between the subject and the imaging unit changed at imaging.

In the second exemplary embodiment, the authentication system is structured so that taking eye images at different distances between the subject and the imaging unit provides at least two eye images under the illuminating rays at different lighting angles to the eye of the subject. In the second exemplary embodiment, elements similar to those in the first exemplary embodiment have the same reference marks, and the descriptions of these elements are omitted.

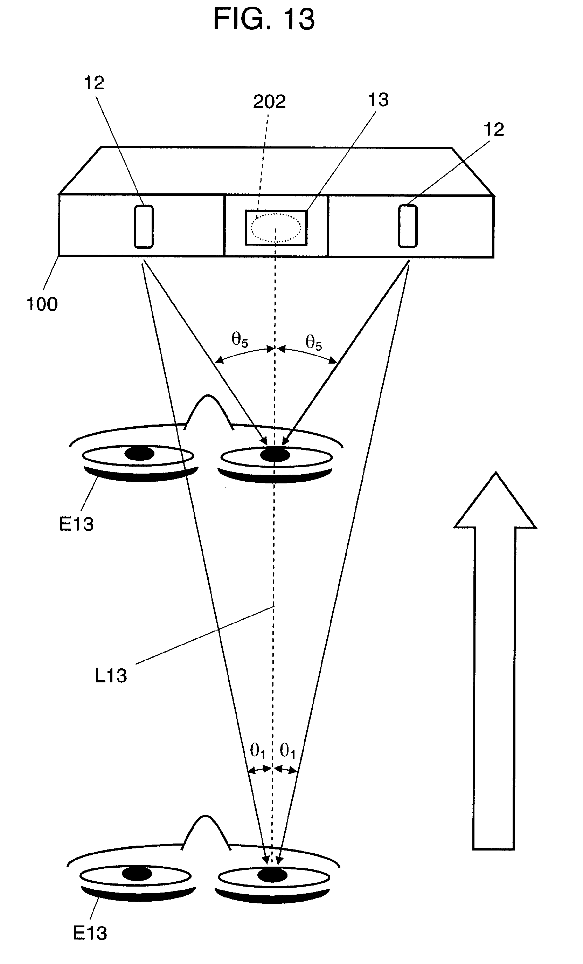

Fig. 13 is a schematic view illustrating authentication system 100 and imaging positions of subject E13 in the second exemplary embodiment of the present invention. With reference to Fig. 13, authentication system 100 includes illuminating LEDs 12, guide mirror 13, and lens 202. As shown in Fig. 13, illuminating LEDs are made of a pair of LEDs disposed symmetrically about the center of lens 202. Authentication system 100 takes eye images in two positions on optical axis L13 of lens 202 in which authentication system 100 and subject E13 are spaced at different distances. Thus, at least two eye images are taken under the illuminating rays at different lighting angles (θ1 and θ5) from illuminating LEDs 12 to the eye of subject E13.

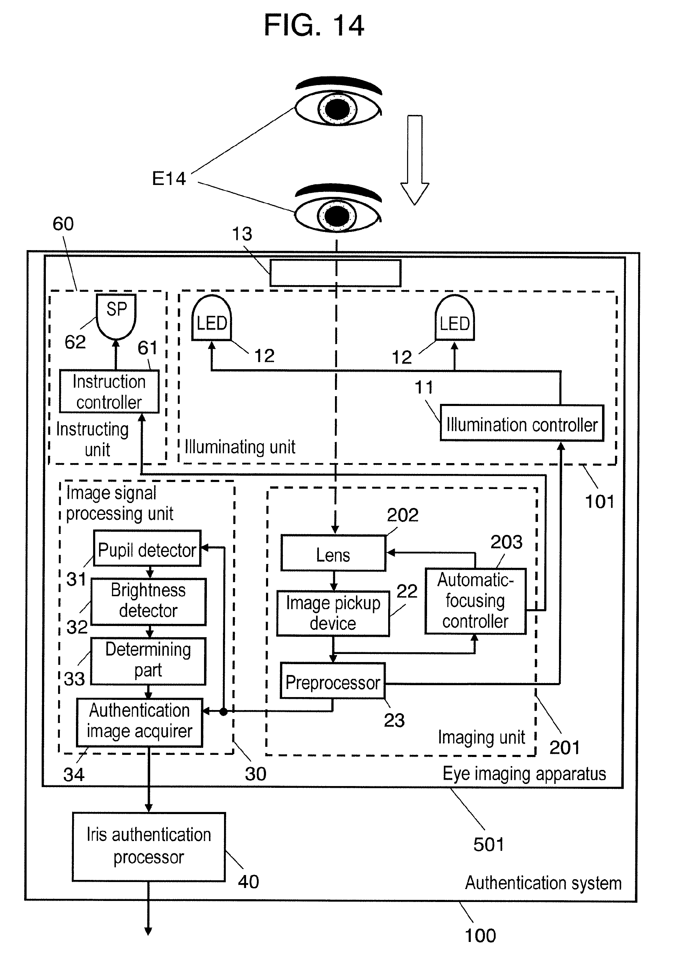

Fig. 14 is a block diagram illustrating structures of eye imaging apparatus 501 and iris authentication processor 40 included in authentication system 100 of the second exemplary embodiment.

As shown in Fig. 14, authentication system 100 of the second exemplary embodiment includes eye imaging apparatus 501 for taking images of eye E14 of a subject, and iris authentication processor 40 for performing authentication operation of the iris using eye images taken in eye imaging apparatus 501.

Eye imaging apparatus 501 includes guide mirror 13, illuminating unit 101, imaging unit 201, image signal processing unit 30, and instructing unit 60.

Guide mirror 13 and image signal processing unit 30 have the same structures and perform the same operation as guide mirror 13 and image signal processing unit 30 of Fig. 6.

Illuminating unit 101 includes illuminating LEDs 12 made of known light-emitting devices radiating near infrared rays, such as a light-emitting diode, and illumination controller 11 for controlling radiation of illuminating LEDs 12. Illuminating LEDs 12 are made of a pair of illuminating LEDs disposed symmetrically about the optical axis of lens 202, and radiate illuminating rays to an area including the eye of the subject. Illumination controller 11 controls the emission luminance of the pair of illuminating LEDs 12 so that a light quantity suitable for eye image acquisition is obtained.

Imaging unit 201 includes commonly used lens 202 having an automatic-focusing function, image pickup device 22, preprocessor 23, and automatic-focusing controller 203 for controlling the automatic focusing function of lens 202. Automatic-focusing controller 203 controls the automatic focusing function of lens 202 using a commonly known method, such as controlling the focusing function so that the differentiated values of the contour portions of the images are minimized, in the eye images taken by image pickup device 22. In the second exemplary embodiment, eye E14 of the subject can be taken in different imaging positions using lens 202 and automatic-focusing controller 203.

Instructing unit 60 includes instruction controller 61 and speaker 62, and instructs the subject of the imaging position by voice. First, instruction controller 61 instructs the subject via speaker 62 to place the eye of the subject in an imaging position in which the illuminating rays are at a small lighting angle of θ1 (e.g. 10°) to the eye of the subject. Then, after the eye image of the subject is taken in that position, the instructing unit instructs the subject via speaker 62 to move to an imaging position in which the lighting angle of the illuminating rays to the eye of the subject is larger than θ1, that is, θ5 (e.g. 50°). The movement of the subject between the imaging positions in this manner can change the lighting angles of the illuminating rays from illuminating LEDs 21 to the eye of the subject.

As described above, in eye imaging apparatus 501 of the second exemplary embodiment of the present invention, imaging the eye of the subject at different distances between the subject and eye imaging apparatus 501 can provide a plurality of eye images taken under illuminating rays at different lighting angles to the eye of the subject.

The major elements for discriminating a living body in the second exemplary embodiment are illuminating unit 101, imaging unit 201, brightness detector 32, and determining part 33. The combinations of these elements are called a biometric discrimination system. The biometric discrimination system of the second exemplary embodiment may further include instructing unit 60 and the entire part of image signal processing unit 30.

The imaging position of the subject can be checked using the amount of control given by automatic-focusing controller 203 to lens 202. However, the eye imaging apparatus may include a distance-measuring sensor, which is commonly known for checking the imaging position of the subject. Alternatively, marks drawn on the ground can inform the subject of the imaging position. In this case, images can be taken by imaging unit 201 at a small lens aperture to increase the depth of field, without the use of the automatic-focusing function.

The instructions may be given from instructing unit 60 to the subject as visual instructions, such as a display device for showing letters, images, or the like, instead of audio instructions using speaker 62.

The function of instruction controller 61 may be implemented by hardware, or described to be implementable by software and implemented by an arithmetic unit or the like. When the function of instruction controller 61 is implemented by software, the instruction controller can be structured of a computer that includes an arithmetic unit having loaded thereon programs for executing the function.

### THIRD EXEMPLARY EMBODIMENT

Next, a description is provided of an authentication system of the third exemplary embodiment of the present invention, with reference to the accompanying drawings.

Described in the first exemplary embodiment is a structure in which illuminating LED parts 12 are made of a plurality of illuminating LEDs, and taking eye images under these LEDs switched for light emission provides a plurality of eye images under illuminating rays at different lighting angles to the eye of the subject. However, the plurality of eye images under illuminating rays at different lighting angles to the eye of the subject can be taken not under illuminating LEDs 12 switched for light emission. Instead, the imaging apparatus has at least two light receivers, and the subject makes a relative movement from an imaging position on the optical axis of one of the light receivers to an imaging position on the optical axis of the other one of the light receivers so that an eye image is taken in each position.

In the third exemplary embodiment of the present invention, at least two eye images under illuminating rays at different lighting angels to the eye of the subject are taken in the following manner. The imaging apparatus has two light receivers, and the subject makes a relative movement from an imaging position on the optical axis of one of the light receivers to an imaging position on the optical axis of the other one of the light receivers so that an eye image is taken in each position. In the third exemplary embodiment, elements similar to those of authentication system 1 of the first exemplary embodiment and authentication system 100 of the second exemplary embodiment have the same reference marks, and the descriptions of these elements are omitted.

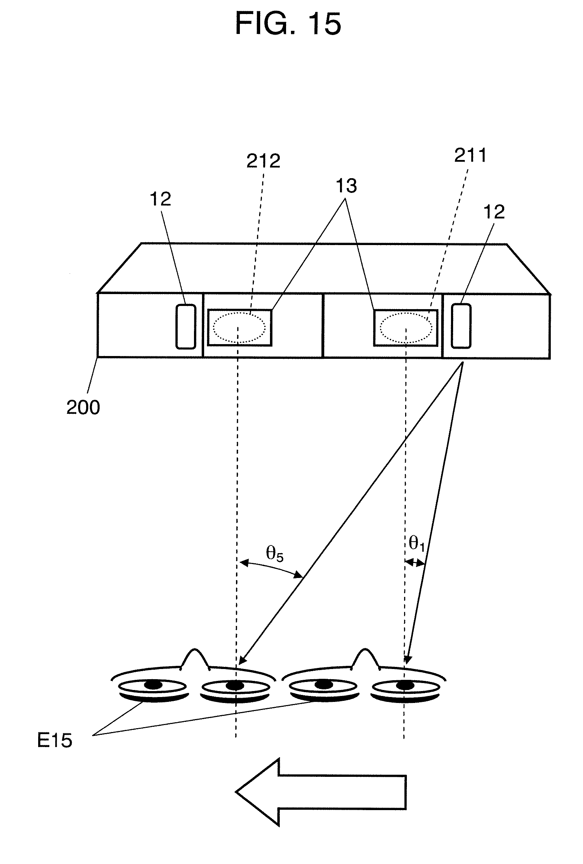

Fig. 15 is a schematic view illustrating authentication system 200 and the imaging positions of subject E15 in the third exemplary embodiment. In Fig. 15, authentication system 200 includes illuminating LEDs 12, guide mirrors 13, and lenses 211 and 212. As shown in Fig. 15, two light receivers included in authentication system 200, i.e. lenses 211 and 212, are disposed so that the respective optical axes are parallel to each other. Illuminating LEDs 12 are made of a pair of LEDs disposed symmetrically outside of lenses 211 and 212. Authentication system 200 takes eye images when an eye of subject E15 is disposed on the axes of lenses 211 and 212, thereby providing at least two eye images under illuminating rays at different lighting angels to the eye of subject E15.

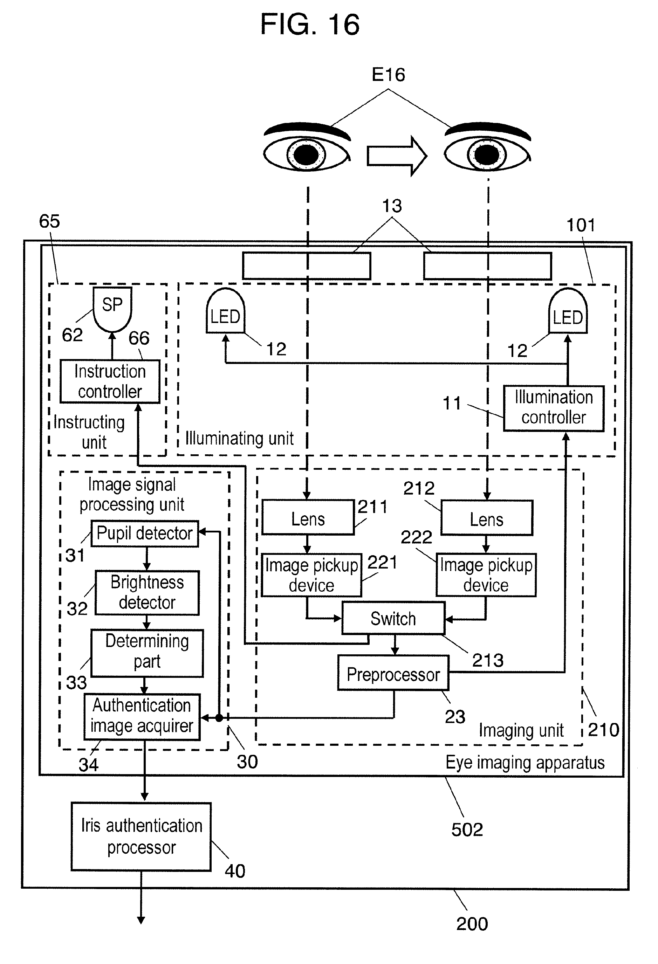

Fig. 16 is a block diagram illustrating structures of eye imaging apparatus 502 and iris authentication processor 40 provided in authentication system 200 in accordance with the third exemplary embodiment.

As shown in Fig. 16, authentication system 200 of the third exemplary embodiment includes eye imaging apparatus 502 for taking eye images of a subject, and iris authentication processor 40 for authenticating the iris using the eye images taken by eye imaging apparatus 502.

Eye imaging apparatus 502 includes guide mirrors 13, illuminating unit 101, imaging unit 210, image signal processing unit 30, and instructing unit 65.

Two guide mirrors 13 are disposed in front of lenses 211 and 212, and perform the same operation as guide mirror 13 of Fig. 6. Image signal processing unit 30 has the same structure and performs the same operation as image signal processing unit 30 of Fig. 6.

Illuminating unit 101 has the same structure and performs the same operation as illuminating unit 101 of Fig. 14.

Imaging unit 210 includes lenses 211 and 212 made of commonly used fixed-focus lenses, image pickup devices 221 and 222 for converting light from lenses 211 and 212 into electrical signals, preprocessor 23, and switch 213 for switching image signals supplied from image pickup devices 221 and 222 and transmitting the image signals to preprocessor 23. Lenses 211 and 212, image pickup devices 221 and 222, and preprocessor 23 have the same structures and perform the same operation as lens 21, image pickup device 22, and preprocessor 23 of Fig. 6. Switch 213 selects image signals supplied from image pickup devices 221 and 222 to transmit to preprocessor 23 the signals from the side where the pupil is detected in pupil detector 31. Switch 213 may switch image signals from lenses 211 and 212 to acquire the image signals on the side to which instructing unit 65 instructs the authenticatee to move in synchronization with instructing unit 65.

Instructing unit 65 includes instruction controller 66 and speaker 62, and instructs the subject of the imaging position by voice. Instruction controller 66 instructs the subject via speaker 62 to place eye E16 of the subject on the optical axis of lens 211. Then, after the image of eye E16 of the subject is taken in that position, the instructing unit instructs the subject via speaker 62 to place the eye of the subject on the optical axis of lens 212. In this manner, an image of eye E16 of the subject is taken on the optical axis of each of lenses 211 and 212.

As described above, eye imaging apparatus 502 of the third exemplary embodiment takes eye images with lenses 211 and 212 having optical axes parallel to each other when the eye of the subject is on one of the optical axes and on the other one of the optical axes, thereby providing a plurality of eye images under illuminating rays at different lighting angles to the eye.

The instructions may be given from instructing unit 65 to the subject as visual instructions, such as a display device for showing letters, images, or the like, instead of audio instructions using speaker 62. Alternatively, light-emitting devices, such as a LED, or other display elements may be provided near guiding mirrors 13 to visually instruct the subject to which side to move.

Each function of instruction controller 66 and switch 213 may be implemented by hardware, or described to be implementable by software and implemented by an arithmetic unit or the like. When each function is implemented by software, each of the instruction controller and switch can be structured of a computer that includes an arithmetic unit having loaded thereon programs for executing the function.

In the description of the third exemplary embodiment, illuminating LEDs 12 are structured of a pair of LEDs. Preferably, the pair of LEDs do not emit rays at the same time, and one of the pair of LEDs emit a ray to illuminate the eye of the subject from only one of the outer side and inner side of the eye. More preferably, the LED illuminates the eye of the subject from the outer side only. The reasons are as follows. When the pair of LEDs emit rays at the same time, the eye of the authenticatee is irradiated with illuminating rays at different lighting angles at the same time. When the eye of the subject is irradiated from the inner side of the eye, the nose of the subject may block the illuminating ray. The following is one of the methods of emitting a ray from one of the pair of LEDs to illuminate the eye of the subject from the outer side of the eye. A subject is required to inputs data on selecting one of the right and left eyes to be used for authentication into authentication system 200, and one of the pair of LEDs is selected to illuminate the selected eye from the outer side thereof. At this time, preferably, brightness detector 32 detects the brightness of the iris and the brightness of the sclera in the taken eye images on the outer side of the eye only.

The major elements for discriminating a living body in the third exemplary embodiment are illuminating unit 101, imaging unit 210, brightness detector 32, and determining part 33. The combinations of these elements are called a biometric discrimination system. The biometric discrimination system of the third exemplary embodiment may further include instructing unit 65 and the entire part of image signal processing unit 30.

Described in the second exemplary embodiment and the third exemplary embodiment are structures in which a plurality of eye images are taken under illuminating rays at different lighting angles to the eye of the subject by the movement of the subject. Similar eye images can be taken by the movement of the imaging apparatus instead of the subject.

### FOURTH EXEMPLARY EMBODIMENT

Next, a description is provided of an authentication system of the fourth exemplary embodiment of the present invention, with reference to the accompanying drawings.

Described in the first exemplary embodiment is a structure in which at least one pair of illuminating LEDs disposed parallel to the optical axis of a lens are switched for light emission, and taking eye images under one pair of LEDs turned on for light emission provides a plurality of eye images under illuminating rays at different lighting angles to the eye of the subject. In other words, the lighting angles of the illuminating rays to the eye of the subject are different between the plurality of eye images taken.

In contrast, irradiating an eye of an authenticatee with at least two illuminating rays in different radiation directions at different lighting angles to the eye of the subject at the same time can provide an image of the eye of the subject under the illuminating rays in at least two different radiation directions at at least two different lighting angels to the eye.

For example, when the eye of the subject is irradiated with illuminating rays in two directions, i.e. from the outer side and the inner side of the eye, the iris in the eye image taken at this time has a difference in brightness between the outer side and the inner side of the eye because the iris of the subject is irradiated with the illuminating rays at different lighting angles on the inner side and the outer side of the eye.

In the fourth exemplary embodiment of the present invention, at least two light-emitting devices are disposed in different radiation directions at different lighting angles to the eye of the subject, and an eye image is taken while the light-emitting devices emit rays at the same time. With this structure, the eye of the subject is imaged while the eye is irradiated with illuminating rays in at least two different radiation directions at at least two different lighting angles at the same time. Thus, one eye image taken allows determination of whether the image is of a living body or a forgery. In the fourth exemplary embodiment, elements similar to those of authentication system 1 in the first exemplary embodiment have the same reference marks, and the descriptions of these elements are omitted.

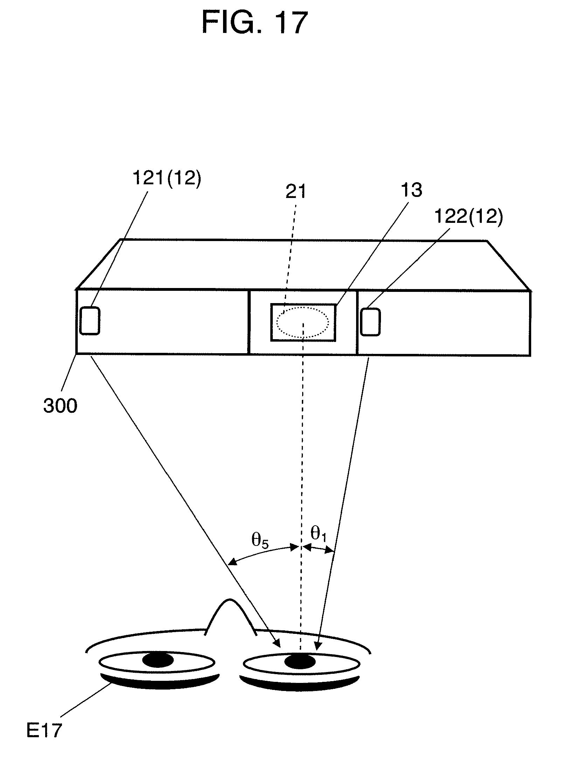

Fig. 17 is a schematic view illustrating authentication system 300 and an imaging position of subject E17 in the fourth exemplary embodiment. With reference to Fig. 17, authentication system 300 includes illuminating LEDs 12, guide mirror 13, and lens 21. As shown in Fig. 17, illuminating LEDs 12 are made of a pair of LEDs 121 and 122 disposed at different distances from the center of lens 21. LEDs 121 and 122 illuminate an eye of subject E17 disposed on the optical axis of lens 21 from the inner side and the outer side of the eye at different lighting angles. Then, authentication system 300 images the eye of subject E 17 while the pair of LEDs 121 and 122 emit rays at the same time. Thus, the authentication system provides an eye image of the subject irradiated with illuminating rays in two different radiation directions at two different lighting angles at the same time.

In the eye image of the subject taken in this manner, the outer side and the inner side of the eye are irradiated with illuminating rays at different lighting angles. In the fourth exemplary embodiment, authentication system 300 calculates an iris contrast, i.e. a brightness ratio between the iris and the sclera, on each of the outer side and the inner side in the taken eye image. Comparison of the calculated contrasts between the outer side and the inner side determines whether the taken eye image is of a living eye or a forgery.

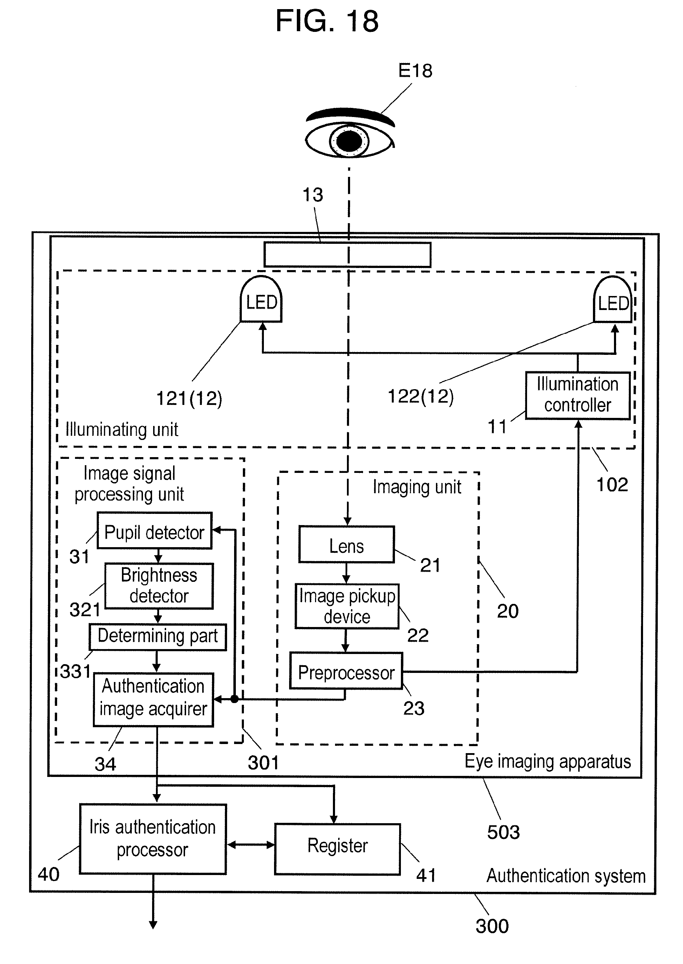

Fig. 18 is a block diagram illustrating structures of eye imaging apparatus 503 and iris authentication processor 40 provided in authentication system 300 of the fourth exemplary embodiment.

As shown in Fig. 18, authentication system 300 of the fourth exemplary embodiment includes eye imaging apparatus 503 for taking an image of eye E18 of the subject to be authenticated, iris authentication processor 40 for authenticating the iris using the eye image taken by eye imaging apparatus 503, and register 41.

Eye imaging apparatus 503 includes guide mirror 13, illuminating unit 102, imaging unit 20, and image signal processing unit 301.

Guide mirror 13 and imaging unit 20 have the same structure and perform the same operation as guide mirror 13 and imaging unit 20 of Fig. 6.

Illuminating unit 102 has the same structure and performs the same operation as illuminating unit 101 of Fig. 14. However, as described above, a pair of LEDs 121 and 122 constituting illuminating LEDs 12 are not disposed symmetrically about the optical axis of lens 21, and are disposed at different distances from the center of lens 21. The illuminating rays emitted from the pair of LEDs 121 and 122 illuminate eye E18 of the subject disposed on the optical axis of lens 21 from the outer side and inner side of the eye at different lighting angles.

Image signal processing unit 301 includes pupil detector 31, brightness detector 321, determining part 331, and authentication image acquirer 34. Pupil detector 31 and authentication image acquirer 34 have the same structure and perform the same operation as pupil detector 31 and authentication image acquirer 34 in image signal processing unit 30 of Fig. 6.

Brightness detector 321 performs similar operation to that of brightness detector 32 in image signal processing unit 30 of Fig. 6. Brightness detector 321 differs from brightness detector 32 in that the brightness is detected on the outer side and the inner side of the eye independently.

Determining part 331 performs similar operation to that of determining part 33 in image signal processing unit 30 of Fig. 6. Determining part 331 differs from determining part 33 in that the brightness on the outer side and the inner side is compared in one taken eye image to determine if the image is of a forgery.

In the fourth exemplary embodiment, the areas in the iris and areas in the sclera for detecting the brightness in the taken eye image are the same as the areas shown in Fig. 8. However, in the fourth exemplary embodiment, the radiation directions of the illuminating rays are not limited. Thus, preferably, appropriate areas are set according to the radiation directions of the illuminating rays from illuminating LEDs 12 to eye E18 of the subject.

As described above, in eye imaging apparatus 503 of the fourth exemplary embodiment, eye E18 of the subject is irradiated with illuminating rays at different lighting angles from the outer side and the inner side of the eye. Then, comparison of the calculated iris contrast between the outer side and the inner side of the eye in one taken eye image can determine whether the taken eye image is of a living eye or a forgery.

The function of image signal processing unit 301 may be implemented by hardware, or described to be implementable by software and implemented by an arithmetic unit or the like. When the function of image signal processing unit 301 is implemented by software, image signal processing unit 301 can be structured of a computer that includes an arithmetic unit having loaded thereon programs for executing the function.

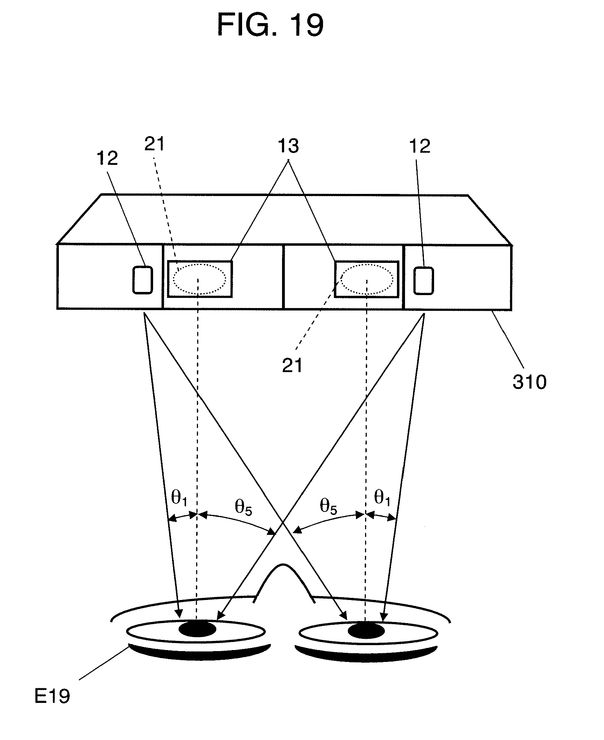

Described in this exemplary embodiment is a structure in which either one of the eyes of a subject is imaged. However, both eyes may be imaged at the same time. Fig. 19 is a schematic view illustrating another example of authentication system 310 and an imaging position of a subject in the fourth exemplary embodiment. Authentication system 310 of Fig. 19 includes two lenses 21 disposed so that the optical axes thereof are parallel to each other, and two guide mirrors 13 disposed in front of lenses 21. The authentication system is capable of imaging both eyes of the subject E19 at the same time. In the structure of Fig. 19, the above determination can be made in the image of both eyes of subject E19. This structure allows more accurate determination of whether the taken eye image is of a living eye or a forgery.

Described in each of the exemplary embodiments of the present invention is a structure in which an eye wearing an artificial eye contact lens is discriminated from the living eye, as an example. Forgeries, such as an artificial eye ball and an imitative photograph, can also be discriminated from a living eye.

Described in each exemplary embodiment of the present invention is a structure in which the iris contrasts are calculated by dividing the brightness of the sclera by the brightness of the iris. Obtaining the difference in brightness between the sclera and the iris in the form of ratios resulting from division can inhibit errors, such as differences in the brightness of the sclera caused by different lighting angles to the illuminating rays, and variations in the luminance and light quantity of illuminating rays in the plurality of taken eye images. However, the present invention is not limited to the structures of these exemplary embodiments.

For example, the emission luminance or light quantity of illuminating LEDs 12 may be controlled in illumination controller 11 so that the brightness of the sclera detected in brightness detector 32 or 321 is constant. In this structure, because the brightness of the sclera in the taken eye images is constant, determining part 33 or 331 can determine if the images are of a forgery only by comparison of the brightness detected in the iris with each other without calculation of the iris contrasts.

Alternatively, preprocessor 23 may perform image processing operation, such as contrast adjustment, on the taken eye images so that the brightness of the sclera detected in brightness detector 32 is constant. Even with such a structure, because the brightness of the sclera in the taken eye images is kept constant, determining part 33 or 331 can determine if the images are of a forgery only by comparison of the brightness detected in the iris with each other without calculation of the iris contrasts.

The major elements for discriminating a living body in the fourth exemplary embodiment are illuminating unit 102, imaging unit 20, brightness detector 32, and determining part 33. The combinations of these elements are called a biometric discrimination system. The biometric discrimination system of the fourth exemplary embodiment may further include the entire part of image signal processing unit 301.

Further, authentication system 300 may include register 41 for registering information on the iris in the eye image determined not to be of a forgery in the biometric discrimination system. Register 41 registers the information on the iris only in the eye image determined not to be of a forgery. Thus, the possibility of registering an unauthorized person by false pretense can be reduced. The location of register 41 is not limited to the inside of authentication system 300. Other authentication system 1, 100, or 200 may include register 41.

Described in each exemplary embodiment of the present invention is a structure in which the illuminating rays from illuminating LEDs 12 are near infrared light. Because irises have characteristics of easily reflecting infrared light, radiation of near infrared light has the following advantages: accuracy of determination improved by a larger difference in iris contrast between a living eye and a forgery; more difficult recognition of the positions of illuminating LEDs 12 emitting rays; and less discomfort felt by the subject when the eye is irradiated. Further, covering illuminating LEDs 12 with commonly known filters for blocking visible light and passing near infrared light can make recognition of the positions of illuminating LEDs 12 emitting rays more difficult.

Described in each exemplary embodiment of the present invention is a structure in which illuminating LEDs 12 are disposed on the right and left sides of the guide mirror. However, illuminating LEDs 12 may be disposed above and below the guide mirror. In this case, preferably, the areas for detecting the brightness of the iris and the brightness of the sclera in the taken eye images are set appropriately according to the radiation directions of the illuminating rays to the subject.

The various kinds of values, including a threshold, described in the exemplary embodiments of the present invention vary with environments in which the eye imaging apparatus is installed, and imaging conditions, such as the luminance and light quantity of illuminating rays. For this reason, preferably, optimal values are obtained by experiments or the like and preset as required.

Further, the authentication system described in each exemplary embodiment of the present invention may include a display device (not shown) using liquid crystal, electroluminescence (EL) or the like for displaying eye images taken by the eye imaging apparatus. When the display device is used by the administrator of the authentication system for confirmation, it is preferable to install the display device distant from the authentication system.

Described in each exemplary embodiment of the present invention is a structure in which the guide mirror is disposed in front of the lens. The lens may be integral with the guide mirror, or disposed near the guide mirror. In each exemplary embodiment of the present invention, the guide mirror is simply used for a subject to understand the horizontal position of the eye or the distance between the eye and the lens. For example, instructions by image or voice may guide the eye of the subject to a proper position, without the use of a guide mirror.

Described in each exemplary embodiment of the present invention is a structure in which a subject is identified as a living body first, and thereafter only when the images of the subject are determined not to be of a forgery, the iris is authenticated. However, for example, the iris of the images may be authenticated first, and thereafter only when the subject is determined to be a registered person, the images may be identified as those of a living body.

Described in each exemplary embodiment of the present invention is a structure in which the eye imaging apparatus and iris authentication processor are integral with each other. However, the present invention is not limited to this structure. Respective components, such as an eye imaging apparatus and iris authentication processor, may be structured as independent components. Alternatively, an eye imaging apparatus may be used for simply taking eye images as a discrete component.

The terms "symmetrically", "equal", and the like are used to express substantial meanings in each exemplary embodiment of the present invention. Within the range in which advantages of the present invention can be E19 maintained, deviation is allowed.

### INDUSTRIAL APPLICABILITY

A biometric discrimination system, an authentication system, and a biometric discrimination method of the present invention have fewer restrictions on the installation site and discriminate forged unauthorized eye images even when an unauthorized person attempts to pretend to be a registered person using a forgery. This structure can decrease the possibility of erroneous registration or authentication of unauthorized eye images. Thus, the present invention is useful as a biometric discrimination system, an authentication system, and a biometric discrimination method.

## Claims

1. A biometric discrimination system comprising:
an illuminating unit for irradiating an area including an eye of a subject with illuminating rays;
an imaging unit for taking a plurality of eye images under the illuminating rays at different lighting angles to the eye of the subject;
a detector for detecting brightness of an iris in the plurality of eye images taken; and
a determining part for comparing the detected brightness of the iris between the plurality of eye images taken and determining whether the subject is a living body or not according to results of the comparison.

2. The biometric discrimination system of claim 1, wherein
the detector detects the brightness of the iris and brightness of a sclera in the plurality of eye images taken; and
the determining part calculates ratios between the detected brightness of the iris and the detected brightness of the sclera, and compares the calculated brightness ratios between the plurality of eye images taken.

3. The biometric discrimination system of claim 2, further including:
a processor for processing the plurality of taken eye images so that the brightness of the sclera to be detected in the detector is constant in the plurality of taken eye images.

4. The biometric discrimination system of claim 2, further including:
a controller for controlling brightness of the illuminating rays, wherein the controller controls the brightness of the illuminating rays so that the brightness of the sclera detected in the detector is constant.

5. The biometric discrimination system of claim 1, wherein
the illuminating unit has a plurality of light-emitting devices disposed at different distances from a predetermined point on an optical axis of the imaging unit and capable of switching for light emission; and
the imaging unit images the eye of the subject irradiated with the illuminating rays at the different lighting angles emitted from the plurality of light emitting devices switched, thereby providing the plurality of eye images under the illuminating rays at the different lighting angles to the eye of the subject.

6. The biometric discrimination system of claim 1, wherein
the imaging unit takes the plurality of eye images of the subject at different distances from the eye on an optical axis of the imaging unit to the imaging unit, thereby providing the plurality of eye images taken under the illuminating rays at the different lighting angles to the eye of the subject.

7. The biometric discrimination system of claim 1, wherein
the imaging unit includes at least two light receivers having optical axes parallel to each other, and imaging the eye of the subject on the respective optical axes of the at least two light receivers provides the plurality of eye images under the illuminating rays at the different lighting angles to the eye of the subject.

8. The biometric discrimination system of claim 6 or 7, further including an instructing unit for instructing the subject to change an imaging position.

9. A biometric discrimination system comprising:
an illuminating unit for irradiating an eye of a subject with at least two illuminating rays in different radiation directions at different lighting angles simultaneously;
an imaging unit for taking an eye image of the subject irradiated with the illuminating rays;
a detector for detecting brightness of at least two different areas in an iris in the taken eye image, the at lest two different areas being preset according to the radiation directions of the illuminating rays to the eye of the subject; and
a determining part for comparing the brightness of the iris detected in the at least two different areas with each other, and determining whether the subject is of a living body or not.

10. The biometric discrimination system of claim 9, wherein
the detector detects brightness of at least two different areas in a sclera set adjacent to the at lest two different areas in the iris; and
the determining part calculates ratios between the brightness of the at least two different areas in the iris and the brightness of the at least two different areas in the sclera in respective adjacent areas, and compares the calculated ratios of the brightness between the different areas.

11. The biometric discrimination system of claim 10, wherein
the illuminating unit radiates the illuminating rays to the eye of the subject disposed on an axis of the imaging unit at the different angles from an outer side and from an inner side of the eye of the subject.

12. The biometric discrimination system of claim 9, further including a controller for controlling brightness of the illuminating rays, wherein
the detector detects brightness of at least two different areas in a sclera set adjacent to the at least two different areas in the iris; and
the controller controls the brightness of the illuminating rays so that the brightness of the at least two different areas in the sclera detected by the detector is constant.

13. The biometric discrimination system of claim 1 or 9, wherein the illuminating unit radiates near infrared rays.

14. An authentication system comprising:
the biometric discrimination system of any one of claims 1, 2, 5, and 9; and
an authentication processor for performing authentication operation using a portion of the iris in the eye images determined not to be of a forgery in the biometric discrimination system.

15. The authentication system of claim 14 further including:
a register for registering information on the iris in the eye images determined not to be of a forgery in the biometric discrimination system.

16. A biometric discrimination method of determining whether or not a subject is a living body using a biometric discrimination system including an illuminating unit, an imaging unit, a detector, and a determining part, the method comprising:
irradiating an eye of the subject with illuminating rays at different lighting angles with the illuminating unit;
taking a plurality of eye images of the subject irradiated with the illuminating rays at the different lighting angles with the imaging unit;
detecting brightness of an iris and brightness of an sclera in the plurality of eye images taken with the detector;
calculating ratios between the detected brightness of the iris and the detected brightness of the sclera with the determining part;
comparing the calculated ratios of the brightness between the plurality of eye images with the determining part; and
determining whether or not the subject is a living body according to a result of the comparison with the determining part.

17. A biometric discrimination method of determining whether or not a subject is a living body using a biometric discrimination system including an illuminating unit, an imaging unit, a detector, and a determining part, the method comprising:
irradiating an eye of the subject with at least two illuminating rays in different radiation directions at different lighting angles simultaneously with the illuminating unit;
taking an eye image of the subject irradiated with the illuminating rays with the imaging unit;
detecting brightness of at least two different areas in an iris in the eye image with the detector, the at least two different areas being preset according to the radiation directions of the illuminating rays to the eye of the subject;
detecting brightness of at least two different areas in a sclera set adjacent to the at least two different areas in the iris with the detector;
calculating ratios between the brightness of the at least two different areas in the iris and the brightness of the at least two different areas in the sclera in respective adjacent areas with the determining part;
comparing the calculated ratios of the brightness with each other in the different areas with the determining part; and
determining whether or not the subject is a living body according to a result of the comparison with the determining part.
